# EUROPEAN PATENT SPECIFICATION

(11) **EP 1 470 148 B1**
(45) Date of publication and mention of the grant of the patent: **18.07.2012**
(21) Application number: 03708949.7
(22) Date of filing: 03.02.2003
(51) Int. Cl.: C12N 15/00

(54) **DOUBLE-STRANDED OLIGONUCLEOTIDES**
DOPPELSTRÄNGIGE OLIGONUKLEOTIDE
OLIGONUCLEOTIDES DOUBLE BRIN

(30) Priority: 01.02.2002 US 353203 P; 01.02.2002 US 353381 P; 23.12.2002 US 436238 P; 07.01.2003 US 438608 P
(43) Date of publication of application: 27.10.2004
(60) Divisional application: 10152725.7
(73) Proprietor: Life Technologies Corporation, Carlsbad, CA 92008 (US)
(72) Inventor: WOOLF, Tod, M., Sudbury, MA 01776 (US); WIEDERHOLT, Kristin, A., Hudson, MA 01749 (US)
(74) Representative: Weber, Birgit
(86) International application number: PCT/US2003/003223
(87) International publication number: WO 2003/064626

(56) References cited:
- US-A- 6 048 974
- US-A- 6 133 024
- PARRISH S ET AL: "Functional Anatomy of a dsRNA trigger: differential requirement for the two trigger strands in RNA interference" MOLECULAR CELL, CELL PRESS, CAMBRIDGE, MA, US, vol. 6, November 2000 (2000-11), pages 1077-1087, XP002226361 ISSN: 1097-2765
- ELBASHIR S M ET AL: "Functional anatomy of siRNAs for mediating efficient RNAi in Drosophila melanogaster embryo lysate" EMBO JOURNAL, OXFORD UNIVERSITY PRESS, SURREY, GB, vol. 20, no. 23, 3 December 2001 (2001-12-03), pages 6877-6888, XP002225998 ISSN: 0261-4189
- KAWASAKI A M ET AL: "UNIFORMLY MODIFIED 2'-DEOXY-2'-FLUORO PHOSPHOROTHIOATE OLIGONUCLEOTIDES AS NUCLEASE-RESISTANT ANTISENSE COMPOUNDS WITH HIGH AFFINITY AND SPECIFICITY FOR RNA TARGETS" JOURNAL OF MEDICINAL CHEMISTRY, AMERICAN CHEMICAL SOCIETY. WASHINGTON, US, vol. 36, no. 7, 2 April 1993 (1993-04-02), pages 831-841, XP000562792 ISSN: 0022-2623
- SERGEEVA Z A ET AL: "Comparative study of modification of DNA and RNA by oligo-(2'-O-methylribonucleotide) derivatives" NUCLEOSIDES & NUCLEOTIDES, MARCEL DEKKER, INC, US, vol. 17, no. 9-11, September 1998 (1998-09), pages 2153-2156, XP009093704 ISSN: 0732-8311
- DE CLERCQ E ET AL: "INTERFERON INDUCTION BY TWO 2' MODIFIED DOUBLE HELICAL RNA POLY 2' FLUORO-2'-DEOXY INOSINIC-ACID POLY CYTIDYLIC-ACID AND POLY-2' CHLORO-2'-DEOXY INOSINIC-ACID POLY CYTIDYLIC-ACID" EUROPEAN JOURNAL OF BIOCHEMISTRY, vol. 107, no. 1, 1980, pages 279-288, XP002462957 ISSN: 0014-2956
- AMARZGUIOUI M ET AL: "Tolerance for mutations and chemical modifications in a siRNA" NUCLEIC ACIDS RESEARCH, OXFORD UNIVERSITY PRESS, SURREY, GB, vol. 31, no. 2, 15 January 2003 (2003-01-15), pages 589-595, XP002270887 ISSN: 0305-1048
- ASHLEY G.W. ET AL.: 'Chemical synthesis of oligodeoxynucleotide dumbbells' BIOCHEMISTRY vol. 30, no. 11, 1991, pages 2927 - 2933, XP002975791
- GIOVANNANGELI C. ET AL.: 'Oligonucleotide clamps arrest DNA synthesis on a single-stranded DNA target' PROC. NATL. ACAD. SCI. vol. 90, November 1993, pages 10013 - 10017, XP002925660
- CHU B.C.F. ET AL.: 'The stability of different forms of double-stranded decoy DNA in serum and nuclear extracts' NUCLEIC ACIDS RESEARCH vol. 20, no. 21, 1992, pages 5857 - 5858, XP000572381
- YAMAKAWA H. ET AL.: 'Properties and anti-hiv activity of nicked dumbbell oligonucleotides' NUCLEOSIDES AND NUCLEOTIDES vol. 15, no. 1-3, 1996, pages 519 - 529, XP002975792

## Description

### Related Applications

This application claims the priority of U.S. provisional patent application no. 60/353,203, filed on February 1, 2002, application no. 60/436,238, filed December 23, 2002, and application no. 60/438,608, filed January 7, 2003. This application also claims the priority of 60/353,381, filed February 1, 2002.

### Background of the Invention

Complementary oligonucleotide sequences are promising therapeutic agents and useful research tools in elucidating gene function. However, oligonucleotide molecules of the prior art are often subject to nuclease degradation when applied to biological systems. Therefore, it is often difficult to achieve efficient inhibition of gene expression (including protein synthesis) using such compositions.

In order to maximize the usefulness, such as the potential therapeutic activity and *in vitro* utility, of oligonucleotides that are complementary to other sequences of interest, it would be of great benefit to improve upon the prior art oligonucleotides by designing improved oligonucleotides having increased stability both against serum nucleases and cellular nucleases and nucleases found in other bodily fluids.

### Summary of the Invention

The instant invention is based, at least in part, on the discovery that double-stranded oligonucleotides comprising an antisense oligonucleotide and a protector oligonucleotide, are capable of inhibiting gene function. Thus, the invention improves the prior art antisense sequences, *inter alia,* by providing oligonucleotides which are resistant to degradation by cellular nucleases.

Accordingly, the invention describes optimized oligonucleotide compositions and methods for making and using both in *in vitro,* and *in vivo* systems, *e.g*., therapeutically. In one aspect the invention provides a method for introducing a double-stranded nucleic acid molecule into a eukaryotic cell in vitro, the method comprising contacting the eukaryotic cell with the double-stranded nucleic acid molecule which comprises a first nucleotide sequence and a second nucleotide sequence which are of equal length;
wherein at least one of the first nucleotide sequence or of the second nucleotide sequence contains at least four 5'-terminal 2'-O-methyl nucleotides, and
wherein the double-stranded nucleic acid molecule is between 18 and 30 nucleotides in length.

In one embodiment the double-stranded nucleic acid molecule is introduced into the eukaryotic cell by electroporation.

In another aspect of the invention a strand of said double-stranded nucleic acid molecule is complementary to a sequence of an mRNA expressed in said eukaryotic cell,

wherein in one embodiment the introduction of said double-stranded nucleic acid molecule in said eukaryotic cell inhibits expression of protein from said mRNA.

In yet another aspect said double-stranded nucleic acid molecule is a double-stranded RNA molecule.

In a further aspect the introduction of said double-stranded RNA molecule in said eukaryotic cell inhibits expression of protein from said mRNA.

Also provided is an embodiment wherein the number of nucleotides in each of the first nucleotide sequence and the second nucleotide sequence of the double-stranded nucleic acid molecule is 21.

In another embodiments the number of nucleotides in each of the first nucleotide sequence and the second nucleotide sequence of the double-stranded nucleic acid molecule is 27 or 26.

In further embodiments, the first nucleotide sequence or the second or both the first and the second nucleotide sequence of the double-stranded nucleic acid molecule contain(s) at least four 5' terminal 2'-O-methyl nucleotides.

### Brief Description of the Drawings

Figure 1 shows that the length of double-stranded oligonucleotides and the presence or absence of overhangs has no effect on function.
Figure 1B shows the effect of structural changes on the efficacy of siRNAs targeting β-3-Integrin.
Figure 2 shows that there is no correlation was observed between the length of the double-stranded oligonucleotide and the level of PKR induction for the given sequences.
Figure 2B shows effect of β-3-integrin targeted 21-mer and 27-mers on PKR expression in HMVEC Cells.
Figure 3 shows the effect of 5' or 3' modification on activity of double-stranded RNA duplexes.
Figure 4 shows the effect of the size of the modifying group on activity of the double-stranded RNA duplex.
Figure 5 shows the results of 2'-O-Me modifications on the activity of double-stranded RNA duplexes.
Figure 6 shows the inhibition of p53 by 32- and 37-mer blunt-end siRNAs.

### Detailed Description of the Invention

The instant invention advances the prior art by providing a method for introducing a double-stranded nucleic and molecule into a eukaryotic cell in vitro.

### Double-stranded Oligonucleotide Compositions

Double-stranded oligonucleotides are capable of inhibiting the synthesis of a target protein, which is encoded by a target gene. The target gene can be endogenous or exogenous (*e.g*., introduced into a cell by a virus or using recombinant DNA technology) to a cell. As used herein, the term "target gene" includes polynucleotides comprising a region that encodes a polypeptide or polynucleotide region that regulates replication, transcription, translation, or other process important in expression of the target protein; or a polynucleotide comprising a region that encodes the target polypeptide and a region that regulates expression of the target polypeptide; or non-coding regions such as the 5' or 3' UTR or introns. Accordingly, the term "target gene" as used herein may refer to, for example, an mRNA molecule produced by transcription a gene of interest. Furthermore, the term "correspond," as in "an oligomer corresponds to a target gene sequence," means that the two sequences are complementary or homologous or bear such other biologically rational relationship to each other (*e.g*., based on the sequence of nucleomonomers and their base-pairing properties).

The "target gene" to which an RNA molecule of the invention is directed may be associated with a pathological condition. For example, the gene may be a pathogen-associated gene, *e.g*., a viral gene, a tumor-associated gene, or an autoimmune disease-associated gene. The target gene may also be a heterologous gene expressed in a recombinant cell or a genetically altered organism. By determining or modulating (*e.g*., inhibiting) the function of such a gene, valuable information and therapeutic benefits in medicine, veterinary medicine, and biology may be obtained.

The term "oligonucleotide" includes two or more nucleomonomers covalently coupled to each other by linkages (*e.g*., phosphodiesters) or substitute linkages. In one embodiment, it may be desirable to use a single-stranded nucleic acid molecule which forms a duplex structure (*e.g*., as described in more detail below). For example, in one embodiment, the oligonucleotide can include a nick in either the sense of the antisense sequence.

The term "antisense" refers to a nucleotide sequence that is inverted relative to its normal orientation for transcription and so expresses an RNA transcript that is complementary to a target gene mRNA molecule expressed within the host cell (*e.g*., it can hybridize to the target gene mRNA molecule through Watson-Crick base pairing). An antisense strand may be constructed in a number of different ways, provided that it is capable of interfering with the expression of a target gene. For example, the antisense strand can be constructed by inverting the coding region (or a portion thereof) of the target gene relative to its normal orientation for transcription to allow the transcription of its complement, (*e.g*., RNAs encoded by the antisense and sense gene may be complementary). Furthermore, the antisense oligonucleotide strand need not have the same intron or exon pattern as the target gene, and noncoding segments of the target gene may be equally effective in achieving antisense suppression of target gene expression as coding segments.

Described herein is a method of inhibiting the activity of a target gene by introducing an RNAi agent into a cell, such that the dsRNA component of the RNAi agent is targeted to the gene. RNA oligonucleotide molecule may contain at least one nucleomonomer that is a modified nucleotide analogue. The nucleotide analogues may be located at positions where the target-specific activity, *e.g*., the RNAi mediating activity is not substantially effected, *e.g*., in a region at the -5'-end or the 3'-end of the double-stranded molecule, where the overhangs may be stabilized by incorporating modified nucleotide analogues.

Double-stranded RNA molecules known in the art can be used in the methods of the present invention. Double-stranded RNA molecules known in the art may also be modified according to the teachings herein in conjunction with such methods, *e.g*., by using modified nucleomonomers. For example, *see* U.S. 6,506,559; U.S. 2002/0,173,478 A1; U.S. 2002/0,086,356 A1; Shuey, et al., "RNAi: gene-silencing in therapeutic intervention." Drug Discov. Today 2002 Oct 15;7(20):1040-6; Aoki, et al., "Clin. Exp. Pharmacol. Physiol. 2003 Jan;30(1-2):96-102; Cioca, et al., "RNA interference is a functional pathway with therapeutic potential in human myeloid leukemia cell lines. Cancer Gene Ther. 2003 Feb;10(2):125-33.

Further examples of double-stranded RNA molecules include those disclosed in the following references: Kawasaki, et al., "Short hairpin type of dsRNAs that are controlled by tRNA(Val) promoter significantly induce RNAi-mediated gene silencing in the cytoplasm of human cells." Nucleic Acids Res. 2003 Jan 15;31(2):700-7; Cottrell, et al., "Silence of the strands: RNA interference in eukaryotic pathogens." Trends Microbiol. 2003 Jan;11(1):37-43; Links, "Mammalian RNAi for the masses." Trends Genet. 2003 Jan;19(1):9-12; Hamada, et al, "Effects on RNA interference in gene expression (RNAi) in cultured mammalian cells of mismatches and the introduction of chemical modifications at the 3'-ends of siRNAs." Antisense Nucleic Acid Drug Dev. 2002 Oct;12(5):301-9; Links, "RNAi and related mechanisms and their potential use for therapy." Curr. Opin. Chem. Biol. 2002 Dec;6(6):829-34; Kawasaki, et al., "Short hairpin type of dsRNAs that are controlled by tRNA(Val) promoter significantly induce RNAi-mediated gene silencing in the cytoplasm of human cells." Nucleic Acids Res. 2003 Jan 15;31(2):700-7.)

A nick is two non-linked nucleomonomers in an oligonucleotide. A nick can be included at any point along the sense or antisense nucleotide sequence. A nick may be in the sense sequence. *e.g*. at least about four nucleomonomers in from an end of the duplexed region of the oligonucleotide (*e.g*., is at least about four nucleomonomers away from the 5' or 3' end of the oligonucleotide or away from a loop structure. For example, the nick may be present in the middle of the sense strand of the duplex molecule (*e.g*., if the sense sequence of the duplex is 30 nucleomonomers in length, nucleomonomers 14 and 15 or 15 and 16 are unlinked). A nick may optionally be ligated to form a circular nucleic acid molecule.

For example, in the structure below, the indicated U nucleomonomer is not bonded to the neighboring nucleomonomer, *e.g*., by a phosphodiester bond. The 5' OH of the nick may optionally be phosphorylated to allow enzymatic ligation of the oligonucleotide into a circle.

A A A 5' G A
G GGCCCUUCUG UCUUGAACAUGAGUU A
A CCGGGAAGACAGAACTTGTACTCAA A
A A G A A A G

(SEQ ID NO: ##)

Figure 5.

As used herein, the term "nucleotide" includes any monomeric unit of DNA or RNA containing a sugar moiety (pentose), a phosphate, and a nitrogenous heterocyclic base. The base is usually linked to the sugar moiety via the glycosidic carbon (at the 1' carbon of pentose) and that combination of base and sugar is called a "nucleoside." The base characterizes the nucleotide with the four customary bases of DNA being adenine (A), guanine (G), cytosine (C) and thymine (T). Inosine (I) is an example of a synthetic base that can be used to substitute for any of the four, naturally-occurring bases (A, C, G, or T). The four RNA bases are A, G, C, and uracil (U). Accordingly, an oligonucleotide may be a nucleotide sequence comprising a linear array of nucleotides connected by phosphodiester bonds between the 3' and 5' carbons of adjacent pentoses. Other modified nucleosides/nucleotides are described herein and may also be used in the methods of the invention.

Oligonucleotides may comprise, for example, oligonucleotides, oligonucleosides, polydeoxyribonucleotides (containing 2'-deoxy-D-ribose) or modified forms thereof, *e.g*., DNA, polyribonucleotides (containing D-ribose or modified forms thereof), RNA, or any other type of polynucleotide which is an N-glycoside or C-glycoside of a purine or pyrimidine base, or modified purine or pyrimidine base. The term oligonucleotide includes compositions in which adjacent nucleomonomers are linked via phosphorothioate, amide or other linkages (*e.g*., Neilsen, P.E., et al. 1991. Science. 254:1497). Generally, the term "linkage" refers to any physical connection, preferably covalent coupling, between two or more nucleic acid components, *e.g*., catalyzed by an enzyme such as a ligase.

In addition to its art-recognized meaning (*e.g*., a relatively short length single or double-stranded sequences of deoxyribonucleotides or ribonucleotides linked via phosphodiester bonds), the term "oligonucleotide" includes any structure that serves as a scaffold or support for the bases of the oligonucleotide, where the scaffold permits binding to the target nucleic acid molecule in a sequence-dependent manner.

Oligonucleotides used in the methods of the invention are isolated. The term "isolated" includes nucleic acid molecules which are synthesized (*e.g*., chemically, enzymatically, or recombinantly) or are naturally occurring but separated from other nucleic acid molecules which are present in a natural source of the nucleic acid. Preferably, a naturally occurring "isolated" nucleic acid molecule is free of sequences which naturally flank the nucleic acid molecule (*i.e*., sequences located at the 5' and 3' ends of the nucleic acid molecule) in a nucleic acid molecule in an organism from which the nucleic acid molecule is derived.

The term "nucleomonomer" includes a single base covalently linked to a second moiety. Nucleomonomers include, for example, nucleosides and nucleotides. Nucleomonomers can be linked to form oligonucleotides that bind to target nucleic acid sequences in a sequence specific manner.

Modified (non-naturally occurring) nucleomonomers can be used in the oligonucleotides described herein. For example, nucleomonomers which are based on bases (purines, pyrimidines, and derivatives and analogs thereof) bound to substituted and unsubstituted cycloalkyl moieties, *e.g.,* cyclohexyl or cyclopentyl moieties, and substituted and unsubstituted heterocyclic moieties, *e.g.,* 6-member morpholino moieties or, preferably, sugar moieties.

Sugar moieties include natural, unmodified sugars, *e.g.,* monosaccharides (such as pentoses, *e.g.,* ribose, deoxyribose), modified sugars and sugar analogs. Possible modifications of nucleomonomers, particularly of a sugar moiety, include, for example, replacement of one or more of the hydroxyl groups with a halogen, a heteroatom, an aliphatic group, or the functionalization of the hydroxyl group as an ether, an amine, a thiol, or the like. One particularly useful group of modified nucleomonomers are 2'-O-methyl nucleotides, especially when the 2'-O-methyl nucleotides are used as nucleomonomers in the ends of the oligomers. Such 2'O-methyl nucleotides may be referred to as "methylated," and the corresponding nucleotides may be made from unmethylated nucleotides followed by alkylation or directly from methylated nucleotide reagents. Modified nucleomonomers may be used in combination with unmodified nucleomonomers. For example, an oligonucleotide of the invention contains at least four methylated nucleomonomers.

Some exemplary modified nucleomonomers include sugar-or backbone-modified ribonucleotides. Modified ribonucleotides may contain a nonnaturally occurring base (instead of a naturally occurring base) such as uridines or cytidines modified at the 5-position, *e.g.,* 5-(2-amino)propyl uridine and 5-bromo uridine; adenosines and guanosines modified at the 8-position, *e.g.,* 8-bromo guanosine; deaza nucleotides, *e.g.,* 7-deaza-adenosine; and N-alkylated nucleotides, *e.g.,* N6-methyl adenosine. Also, sugar-modified ribonucleotides may have the 2'-OH group replaced by a H, alxoxy (or OR), R or alkyl, halogen, SH, SR, amino (such as NH₂, NHR, NR₂,), or CN group, wherein R is lower alkyl, alkenyl, or alkynyl.

Modified ribonucleotides may also have the phosphoester group connecting to adjacent ribonucleotides replaced by a modified group, *e.g.,* of phosphothioate group. More generally, the various nucleotide modifications may be combined.

Sense oligomers may have 2' modifications on the ends (1 on each end, 2 on each end, 3 on each end, and 4 on each end, and so on; as well as 1 on one end, 2 on one end, 3 on one end, and 4 on one end, and so on; and even unbalanced combinations such as 1 on one end and 2 on the other end, and so on). Likewise, the antisense strand may have 2' modifications on the ends (1 on each end, 2 on each end, 3 on each end, and 4 on each end, and so on; as well as 1 on one end, 2 on one end, 3 on one end, and 4 on one end, and so on; and even unbalanced combinations such as 1 on one end and 2 on the other end, and so on). Such 2'-modifications may be in the sense RNA strand or the sequences other than the antisense strand.

To further maximize endo- and exonuclease resistance, in addition to the use of 2' modified nucleomonomers in the ends, inter-nucleomonomer linkages other than phosphodiesters may be used. For example, such end blocks may be used alone or in conjunction with phosphothorothioate linkages between the 2'-O-methly linkages. Preferred 2'-modified nucleomonomers are 2'-modified C and U bases.

Although the antisense strand may be substantially identical to at least a portion of the target gene (or genes), at least with respect to the base pairing properties, the sequence need not be perfectly identical to be useful, *e.g*., to inhibit expression of a target gene's phenotype. Generally, higher homology can be used to compensate for the use of a shorter antisense gene. In some cases, the antisense strand generally will be substantially identical (although in antisense orientation) to the target gene.

One particular example of a composition has end-blocks on both ends of a sense oligonucleotide and only the 3' end of an antisense oligonucleotide. Without wishing to be bound by theory, the inventors believe that a 2'-O-modified sense strand works less well than unmodified because it is not efficiently unwound. Accordingly, another example includes duplexes in which nucleomonomer-nucleomonomer mismatches are present in a sense 2'-O-methly strand (and are thought to be easier to unwind).

Accordingly, for a given first oligonucleotide strand, a number of complementary second oligonucleotide strands are permitted. For example, in the following Tables, a targeted and a non-targeted oligonucleotide are illustrated with several possible complementary oligonucleotides. The individual nucleotides may be 2'-OH RNA nucleotides (R) or the corresponding 2'-OMe nucleotides (M), and the oligonucleotides themselves may contain mismatched nucleotides (lower case letters).

### Targeted Oligonucleotide:

| | | |
|---|---|---|
| First Strand: | CCCUUCUGUCUUGAACAUGAG | (SEQ ID NO: ##) |
| Second Strand: (methyl groups →) | CTgATGTTCAAGACAGAAcGG MMMMMMMMMMMMMMMMMMMMM | (SEQ ID NO: ##) |
| | CTgATGTTCAAGACAGAAcGG RRRRRRRRRRRRRRRRRRRDD | (SEQ ID NO: ##) |
| | CTCAUGUUCAAGACAGAAGGG RRRRRRMMMMMMMMMRRRRRR | (SEQ ID NO: ##) |
| | CTCAUGUUCAAGACAGAAGGG MMMMMMRRRRRRRRRMMMMMM | (SEQ ID NO: ##) |
| | CTCAUGUUCAAGACAGAAGGG RMRMRMRMRMRMRMRMRMRMR | (SEQ ID NO: ##) |

### Non-Targeted Oligonucleotide:

| | | |
|---|---|---|
| First Strand: | GAGTACAAGTTCTGTCTTCCC | (SEQ ID NO: ##) |
| Second Strand: (methyl groups →) | GGcAAGACAGAACTTGTAgTC MMMMMMMMMMMMMMMMMMMMM | (SEQ ID NO: ##) |
| | GGGAAGACAGAACTTGTACTC RRRRRRMMMMMMMMMRRRRRR | (SEQ ID NO: ##) |
| | GGGAAGACAGAACTTGTACTC MMMMMMRRRRRRRRRMMMMMM | (SEQ ID NO: ##) |
| | GGGAAGACAGAACTTGTACTC RMRMRMRMRMRMRMRMRMRMR | (SEQ ID NO: ##) |

Another example of further modifications that may be used in conjunction with 2'-O-methyl nucleomonomers are modification of the sugar residues themselves, for example alternating modified and unmodified sugars particularly in the sense strand.

In one embodiment, of the method of the invention the length of the sense strand can be 29, 28, 27, 26, 25, 24, 23, 22, 21, 20, 19, or 18 nucleotides, with a complementary duplexed RNA strand,

As a further example, the use of 2'-O-methyl RNA may beneficially be used in circumstances in which it is desirable to minimize cellular stress responses. RNA having 2'-O-methyl nucleomonomers may not be recognized by cellular machinery that is thought to recognize unmodified RNA. The use of 2'-O-methylated or partially 2'-O-methylated RNA may avoid the interferon response to double-stranded nucleic acids, while maintaining target RNA inhibition. This RNAi ("stealth RNAi") is useful for avoiding the interferon or other cellular stress responses, both in short RNAi (*e.g*., siRNA) sequences that induce the interferon response, and in longer RNAi sequences that may induce the interferon response.

The method of the present invention may be used in gene function studies in which multiple RNAi sequences are used. According to present methods known in the art, frequently there is no way of predicting which sequences might induce a stress response, including the interferon response, and in this regard the present invention significantly advances the state of the art. For example, if all of the multiple sequences are partially 2-O-methylated, the stress response, including interferon response, may be avoided, and thus avoid confounding results in which some sequences affect cellular phenotype independent of the target gene inhibition. Other chemical modifications in addition to 2'-O-methylation may also achieve this effect.

For example, modified sugars include D-ribose, 2'-O-alkyl (including 2'-O-methyl and 2'-O-ethyl), *i.e.,* 2'-alkoxy, 2'-amino, 2'-S-alkyl, 2'-halo (including 2'-fluoro), 2'-methoxyethoxy, 2'-allyloxy (-OCH₂CH=CH₂), 2'-propargyl, 2'-propyl, ethynyl, ethenyl, propenyl, and cyano and the like. In one embodiment, the sugar moiety can be a hexose and incorporated into an oligonucleotide as described (Augustyns, K., et al., Nucl. Acids. Res. 1992. 18:4711). Exemplary nucleomonomers can be found, *e.g.,* in U.S. Patent 5,849,902, .

The term "alkyl" includes saturated aliphatic groups, including straight-chain alkyl groups (*e.g*., methyl, ethyl, propyl, butyl, pentyl, hexyl, heptyl, octyl, nonyl, decyl, *etc*.), branched-chain alkyl groups (isopropyl, tert-butyl, isobutyl, *etc.*), cycloalkyl (alicyclic) groups (cyclopropyl, cyclopentyl, cyclohexyl, cycloheptyl, cyclooctyl), alkyl substituted cycloalkyl groups, and cycloalkyl substituted alkyl groups. In certain embodiments, a straight chain or branched chain alkyl has 6 or fewer carbon atoms in its backbone (*e.g*., C₁-C₆ for straight chain, C₃-C₆ for branched chain), and more preferably 4 or fewer. Likewise, preferred cycloalkyls have from 3-8 carbon atoms in their ring structure, and more preferably have 5 or 6 carbons in the ring structure. The term C₁-C₆ includes alkyl groups containing 1 to 6 carbon atoms.

Moreover, unless otherwise specified, the term alkyl includes both "unsubstituted alkyls" and "substituted alkyls," the latter of which refers to alkyl moieties having substituents replacing a hydrogen on one or more carbons of the hydrocarbon backbone. Such substituents can include, for example, alkenyl, alkynyl, halogen, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxyl, phosphate, phosphonato, phosphinato, cyano, amino (including alkyl amino, dialkylamino, arylamino, diarylamino, and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkylaryl, or an aromatic or heteroaromatic moiety. Cycloalkyls can be further substituted, *e.g.,* with the substituents described above. An "alkylaryl" or an "arylalkyl" moiety is an alkyl substituted with an aryl (*e.g*., phenylmethyl (benzyl)). The term "alkyl" also includes the side chains of natural and unnatural amino acids. The term "*n*-alkyl" means a straight chain (*i.e.,* unbranched) unsubstituted alkyl group.

The term "alkenyl" includes unsaturated aliphatic groups analogous in length and possible substitution to the alkyls described above, but that contain at least one double bond. For example, the term "alkenyl" includes straight-chain alkenyl groups (*e.g*., ethylenyl, propenyl, butenyl, pentenyl, hexenyl, heptenyl, octenyl, nonenyl, decenyl, *etc*.), branched-chain alkenyl groups, cycloalkenyl (alicyclic) groups (cyclopropenyl, cyclopentenyl, cyclohexenyl, cycloheptenyl, cyclooctenyl), alkyl or alkenyl substituted cycloalkenyl groups, and cycloalkyl or cycloalkenyl substituted alkenyl groups. In certain embodiments, a straight chain or branched chain alkenyl group has 6 or fewer carbon atoms in its backbone (*e.g.*, C₂-C₆ for straight chain, C₃-C₆ for branched chain). Likewise, cycloalkenyl groups may have from 3-8 carbon atoms in their ring structure, and more preferably have 5 or 6 carbons in the ring structure. The term C₂-C₆ includes alkenyl groups containing 2 to 6 carbon atoms.

Moreover, unless otherwise specified, the term alkenyl includes both "unsubstituted alkenyls" and "substituted alkenyls," the latter of which refers to alkenyl moieties having substituents replacing a hydrogen on one or more carbons of the hydrocarbon backbone. Such substituents can include, for example, alkyl groups, alkynyl groups, halogens, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxyl, phosphate, phosphonato, phosphinato, cyano, amino (including alkyl amino, dialkylamino, arylamino, diarylamino, and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkylaryl, or an aromatic or heteroaromatic moiety.

The term "alkynyl" includes unsaturated aliphatic groups analogous in length and possible substitution to the alkyls described above, but which contain at least one triple bond. For example, the term "alkynyl" includes straight-chain alkynyl groups (*e.g*., ethynyl, propynyl, butynyl, pentynyl, hexynyl, heptynyl, octynyl, nonynyl, decynyl, *etc*.), branched-chain alkynyl groups, and cycloalkyl or cycloalkenyl substituted alkynyl groups. In certain embodiments, a straight chain or branched chain alkynyl group has 6 or fewer carbon atoms in its backbone (*e.g*., C₂-C₆ for straight chain, C₃-C₆ for branched chain). The term C₂-C₆ includes alkynyl groups containing 2 to 6 carbon atoms.

Moreover, unless otherwise specified, the term alkynyl includes both "unsubstituted alkynyls" and "substituted alkynyls," the latter of which refers to alkynyl moieties having substituents replacing a hydrogen on one or more carbons of the hydrocarbon backbone. Such substituents can include, for example, alkyl groups, alkynyl groups, halogens, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxyl, phosphate, phosphonato, phosphinato, cyano, amino (including alkyl amino, dialkylamino, arylamino, diarylamino, and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkylaryl, or an aromatic or heteroaromatic moiety.

Unless the number of carbons is otherwise specified, "lower alkyl" as used herein means an alkyl group, as defined above, but having from one to five carbon atoms in its backbone structure. "Lower alkenyl" and "lower alkynyl" have chain lengths of, for example, 2-5 carbon atoms.

The term "alkoxy" includes substituted and unsubstituted alkyl, alkenyl, and alkynyl groups covalently linked to an oxygen atom. Examples of alkoxy groups include methoxy, ethoxy, isopropyloxy, propoxy, butoxy, and pentoxy groups. Examples of substituted alkoxy groups include halogenated alkoxy groups. The alkoxy groups can be substituted with groups such as alkenyl, alkynyl, halogen, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxyl, phosphate, phosphonato, phosphinato, cyano, amino (including alkyl amino, dialkylamino, arylamino, diarylamino, and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkylaryl, or an aromatic or heteroaromatic moieties. Examples of halogen substituted alkoxy groups include, but are not limited to, fluoromethoxy, difluoromethoxy, trifluoromethoxy, chloromethoxy, dichloromethoxy, trichloromethoxy, *etc*.

The term "heteroatom" includes atoms of any element other than carbon or hydrogen. Preferred heteroatoms are nitrogen, oxygen, sulfur and phosphorus.

The term "hydroxy" or "hydroxyl" includes groups with an -OH or -O⁻ (with an appropriate counterion).

The term "halogen" includes fluorine, bromine, chlorine, iodine, etc. The term "perhalogenated" generally refers to a moiety wherein all hydrogens are replaced by halogen atoms.

The term "substituted" includes substituents which can be placed on the moiety and which allow the molecule to perform its intended function. Examples of substituents include alkyl, alkenyl, alkynyl, aryl, (CR'R")₀₋₃NR'R", (CR'R")₀₋₃CN, NO₂, halogen, (CR'R")₀₋₃C(halogen)₃, (CR'R")₀₋₃CH(halogen)₂, (CR'R")₀₋₃CH₂(halogen), (CR'R")₀₋₃CONR'R", (CR'R")₀₋₃S(O)₁₋₂NR'R", (CR'R")₀₋₃CHO, (CR'R")₀₋₃O(CR'R")₀₋₃H, (CR'R")₀₋₃S(O)₀₋₂R', (CR'R")₀₋₃O(CR'R")₀₋₃H, (CR'R")₀₋₃COR', (CR'R")₀₋₃CO₂R', or (CR'R")₀₋₃0R' groups; wherein each R' and R" are each independently hydrogen, a C₁-C₅ alkyl, C₂-C₅ alkenyl, C₂-C₅ alkynyl, or aryl group, or R' and R" taken together are a benzylidene group or a -(CH₂)₂O(CH₂)₂- group.

The term "amine" or "amino" includes compounds or moieties in which a nitrogen atom is covalently bonded to at least one carbon or heteroatom. The term "alkyl amino" includes groups and compounds wherein the nitrogen is bound to at least one additional alkyl group. The term "dialkyl amino" includes groups wherein the nitrogen atom is bound to at least two additional alkyl groups.

The term "ether" includes compounds or moieties which contain an oxygen bonded to two different carbon atoms or heteroatoms. For example, the term includes "alkoxyalkyl," which refers to an alkyl, alkenyl, or alkynyl group covalently bonded to an oxygen atom which is covalently bonded to another alkyl group.

The term "base" includes the known purine and pyrimidine heterocyclic bases, deazapurines, and analogs (including heterocyclic substituted analogs, e.g., aminoethyoxy phenoxazine), derivatives (*e.g*., 1-alkyl-, 1-alkenyl-, heteroaromatic- and 1-alkynyl derivatives) and tautomers thereof. Examples of purines include adenine, guanine, inosine, diaminopurine, and xanthine and analogs (*e.g*., 8-oxo-N⁶-methyladenine or 7-diazaxanthine) and derivatives thereof. Pyrimidines include, for example, thymine, uracil, and cytosine, and their analogs (*e.g*., 5-methylcytosine, 5-methyluracil, 5-(1-propynyl)uracil, 5-(1-propynyl)cytosine and 4,4-ethanocytosine). Other examples of suitable bases include non-purinyl and non-pyrimidinyl bases such as 2-aminopyridine and triazines.

In a preferred embodiment, of the method of the invention the double-stranded nucleic acid molecule is a double-stranded RNA molecule. In another preferred embodiment, the nucleomonomers are modified RNA nucleotides.

The term "nucleoside" includes bases which are covalently attached to a sugar moiety, preferably ribose or deoxyribose. Examples of nucleosides include ribonucleosides and deoxyribonucleosides. Nucleosides also include bases linked to amino acids or amino acid analogs which may comprise free carboxyl groups, free amino groups, or protecting groups. Suitable protecting groups are well known in the art (*see* P.G.M. Wuts and T.W. Greene, "Protective Groups in Organic Synthesis", 2nd Ed., Wiley-Interscience, New York, 1999).

The term "nucleotide" includes nucleosides which further comprise a phosphate group or a phosphate analog.

As used herein, the term "linkage" includes a naturally occurring, unmodified phosphodiester moiety (-O-(PO₂⁻)-O-) that covalently couples adjacent nucleomonomers. As used herein, the term "substitute linkage" includes any analog or derivative of the native phosphodiester group that covalently couples adjacent nucleomonomers. Substitute linkages include phosphodiester analogs, *e.g.,* phosphorothioate, phosphorodithioate, and P-ethyoxyphosphodiester, P-ethoxyphosphodiester, P-alkyloxyphosphotriester, methylphosphonate, and nonphosphorus containing linkages, *e.g.,* acetals and amides. Such substitute linkages are known in the art (*e.g*., Bjergarde et al. 1991. Nucleic Acids Res. 19:5843; Caruthers et al. 1991. Nucleosides Nucleotides. 10:47).

Oligonucleotides comprise 3' and 5' termini (except for circular oligonucleotides). The 3' and 5' termini of an oligonucleotide can be substantially protected from nucleases *e.g*., by modifying the 3' or 5' linkages (*e.g*., U.S. patent 5,849,902 and WO 98/13526). For example, oligonucleotides can be made resistant by the inclusion of a "blocking group." The term "blocking group" as used herein refers to substituents (*e.g*., other than OH groups) that can be attached to oligonucleotides or nucleomonomers, either as protecting groups or coupling groups for synthesis (*e.g*., FITC, propyl (CH₂-CH₂-CH₃), phosphate (PO₃²⁻), hydrogen phosphonate, or phosphoramidite). "Blocking groups" also include "end blocking groups" or "exonuclease blocking groups" which protect the 5' and 3' termini of the oligonucleotide, including modified nucleotides and non-nucleotide exonuclease resistant structures.

Exemplary end-blocking groups include cap structures (*e.g*., a 7-methylguanosine cap), inverted nucleomonomers, *e.g*., with 3'-3' or 5'-5' end inversions (*see, e.g.,* Ortiagao et al. 1992. Antisense Res. Dev. 2:129), methylphosphonate, phosphoramidite, non-nucleotide groups (*e.g*., non-nucleotide linkers, amino linkers, conjugates) and the like. The 3' terminal nucleomonomer can comprise a modified sugar moiety. The 3' terminal nucleomonomer comprises a 3'-O that can optionally be substituted by a blocking group that prevents 3'-exonuclease degradation of the oligonucleotide. For example, the 3'-hydroxyl can be esterified to a nucleotide through a 3'→3' internucleotide linkage. For example, the alkyloxy radical can be methoxy, ethoxy, or isopropoxy, and preferably, ethoxy. Optionally, the 3'→3' linked nucleotide at the 3' terminus can be linked by a substitute linkage. To reduce nuclease degradation, the 5' most 3'→5' linkage can be a modified linkage, *e.g.,* a phosphorothioate or a P-alkyloxyphosphotriester linkage. 5'-Optionally, the 5' terminal hydroxy moiety can be esterified with a phosphorus containing moiety, *e.g.,* phosphate, phosphorothioate, or P-ethoxyphosphate.

The sense strand of an oligonucleotide may comprise a 5' group that allows for RNAi activity but which renders the sense strand inactive in terms of gene targeting. Such a 5' modifying group may be a phosphate group or a group larger than a phosphate group

The antisense strand of an oligonucleotide can comprise a 5' phosphate group.

The oligonucleotides may be high affinity oligonucleotides. The term "high affinity" as used herein includes oligonucleotides that have a Tm (melting temperature) of or greater than about 60°C, greater than about 65°C, greater than about 70°C, greater than about 75°C, greater than about 80 °C or greater than about 85 °C. The Tm is the midpoint of the temperature range over which the oligonucleotide separates from the target nucleotide sequence. At this temperature, 50% helical (hybridized) versus coil (unhybridized) forms are present. Tm is measured by using the UV spectrum to determine the formation and breakdown (melting) of hybridization. Base stacking occurs during hybridization, which leads to a reduction in UV absorption. Tm depends both on GC content of the two nucleic acid molecules and on the degree of sequence complementarity. Tm can be determined using techniques that are known in the art (see for example, Monia et al. 1993. J. Biol. Chem. 268:145; Chiang et al. 1991. J. Biol. Chem. 266:18162; Gagnor et al. 1987. Nucleic Acids Res. 15:10419; Monia et al. 1996. Proc. Natl. Acad. Sci. 93:15481; Publisis and Tinoco. 1989. Methods in Enzymology 180:304; Thuong et al. 1987. Proc. Natl. Acad. Sci. USA 84:5129).

An oligonucleotide can include an agent which increases the affinity of the oligonucleotide for its target sequence. The term "affinity enhancing agent" includes agents that increase the affinity of an oligonucleotide for its target. Such agents include, *e.g*., intercalating agents and high affinity nucleomonomers. Intercalating agents interact strongly and nonspecifically with nucleic acids. Intercalating agents serve to stabilize RNA-DNA duplexes and thus increase the affinity of the oligonucleotides for their targets. Intercalating agents are most commonly linked to the 3' or 5' end of oligonucleotides. Examples of intercalating agents include acridine, chlorambucil, benzopyridoquinoxaline, benzopyridoindole, benzophenanthridine, and phenazinium. The agents may also impart other characteristics to the oligonucleotide, for example, increasing resistance to endonucleases and exonucleases.

The language "high affinity nucleomonomer" as used herein includes modified bases or base analogs that bind to a complementary base in a target nucleic acid molecule with higher affinity than an unmodified base, for example, by having more energetically favorable interactions with the complementary base, *e.g*., by forming more hydrogen bonds with the complementary base. For example, high affinity nucleomonomer analogs such as aminoethyoxy phenoxazine (also referred to as a G clamp), which forms four hydrogen bonds with guanine are included in the term "high affinity nucleomonomer." A high affinity nucleomonomer is illustrated below (*see, e.g.,* Flanagan, et al., 1999. Proc. Natl. Acad. Sci. 96:3513).

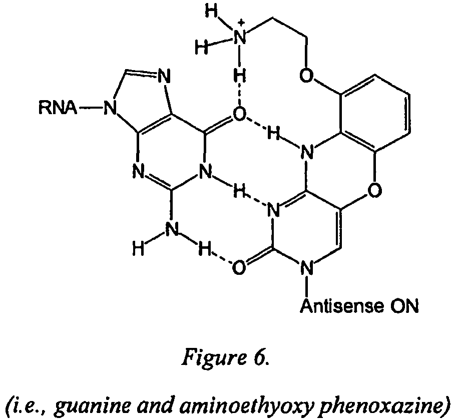

Other exemplary high affinity nucleomonomers are known in the art and include 7-alkenyl, 7-alkynyl, 7-heteroaromatic-, or 7-alkynyl-heteroaromatic-substituted bases or the like which can be substituted for adenosine or guanosine in oligonucleotides (*see, e.g.,* U.S. patent 5,594,121). Also, 7-substituted deazapurines have been found to impart enhanced binding properties to oligonucleotides, *i.e*., by allowing them to bind with higher affinity to complementary target nucleic acid molecules as compared to unmodified oligonucleotides. High affinity nucleomonomers can be incorporated into the oligonucleotides of the instant invention using standard techniques.

As used herein, the language "intercalating agent" includes agents which can bind to a DNA double helix. An intercalating agent enhances the binding of the oligonucleotide to its complementary genomic DNA target sequence. The intercalating agent may also increase resistance to endonucleases and exonucleases.

Exemplary intercalating agents are taught by Helene and Thuong (1989. Genome 31:413), and include *e.g*., acridine derivatives (Lacoste et al. 1997. Nucleic Acids Research. 25:1991; Kukreti et al. 1997. Nucleic Acids Research. 25:4264); quinoline derivatives (Wilson et al. 1993. Biochemistry 32:10614); benzo[f]quino[3,4-b]quioxaline derivatives (Marchand et al. 1996. Biochemistry. 35:5022; Escude et al. 1998. Proc. Natl. Acad. Sci. 95:3591).

Intercalating agents can be incorporated into an oligonucleotide using any convenient linkage. For example, acridine or psoralen can be linked to the oligonucleotide through any available -OH or -SH group, *e.g*., at the terminal 5' position of the oligonucleotide, the 2' positions of sugar moieties, or an OH, NH₂, COOH, or SH incorporated into the 5-position of pyrimidines using standard methods.

When included in an RNase H activating antisense nucleotide sequence, an agent that increases the affinity of an oligonucleotide for its target is not positioned adjacent to an RNase activating region of the oligonucleotide, *e.g.,* is positioned adjacent to a non-RNase activating region. Preferably, the agent that increases the affinity of an oligonucleotide for its target is placed at a distance as far as possible from the RNase activating domain of the chimeric antisense sequence such that the specificity of the chimeric antisense sequence is not altered when compared with the specificity of a chimeric antisense sequence which lacks the intercalating compound. This can be accomplished by positioning the agent adjacent to a non-RNase activating region. The specificity of the oligonucleotide can be tested by demonstrating that transcription of a non-target sequence, preferably a non-target sequence which is structurally similar to the target (*e.g*., has some sequence homology or identity with the target sequence but which is not identical in sequence to the target), is not inhibited to a greater degree by an oligonucleotide comprising an affinity enhancing agent than by an oligonucleotide directed against the same target that does not comprise an affinity enhancing agent.

The double-stranded oligonucleotides may be formed by a single, self-complementary nucleic acid strand or two separate complementary nucleic acid strands.

As used herein, the term "double-stranded" includes one or more nucleic acid molecules comprising a region of the molecule in which at least a portion of the nucleomonomers are complementary and hydrogen bond to form a duplex.

As used herein, the term "duplex" includes the region of the double-stranded nucleic acid molecule(s) that is (are) hydrogen bonded to a complementary sequence.

The double-stranded nucleic acids to be used in the method invention comprise a nucleotide sequence that is sense to a target gene and a complementary sequence that is antisense to the target gene. The sense and antisense nucleotide sequences correspond to the target gene sequence, e.g., are identical or are sufficiently identical to effect target gene inhibition (*e.g*., are about at least about 98%, 96% identical, 94%, 90% identical, 85% identical, or 80% identical) to the target gene sequence.

In one embodiment, a double-stranded oligonucleotide is double-stranded over its entire length, *i.e.,* with no overhanging single-stranded sequence at either end of the molecule, *i.e.,* is blunt-ended. In another example a double-stranded oligonucleotide is not double-stranded over its entire length.

A double-stranded oligonucleotide may be double-stranded over at least about 70% of the length of the oligonucleotide or double-stranded over at least about 80% of the length of the oligonucleotide or double-stranded over at least about 90%-95% of embodiment, a double or over at least about 96%-98% of the length of the oligonucleotide.

The double-stranded duplex constructs can be further stabilized against nucleases by forming loop structures at the 5' or 3' end of the sense or antisense strand of the construct. For example, the construct.can take the form: 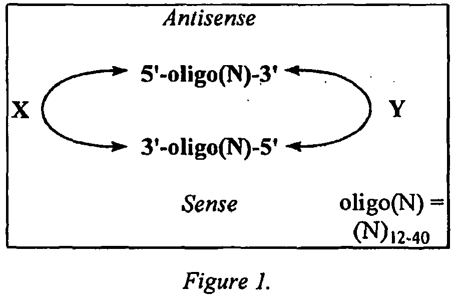 where the Ns are nucleomonomers in complementary oligonucleotide strands (*i.e*., the top N strand is complementary to the bottom N strand) of equal length (*e.g*., between about 12 and about 40 nucleotides in length) and X and Y are each independently selected from a group consisting of nothing (*i.e.*, the construct is a blunt ended construct with no loops and no overhang); from about 1 to about 20 nucleotides of 5' overhang; from about 1 to about 20 nucleotides of 3' overhang; a GAAA loop (tetra-loop); and a loop consisting from about 4 to about 20 nucleomonomers (where the nucleomonomers are all either Gs or A's).

The sequence of Ns corresponds to the target gene sequence (*e.g*., is homologous or identical to a nucleotide sequence that is sense or antisense to the target gene sequence), while the nucleotide sequence of the loop structure does not correspond to the target gene sequence.

For example, such loops can comprise all Gs and A's and be from about 4 to about 20 nucleotides in length. Such a loop can be a tetra-loop having a sequence GAAA:

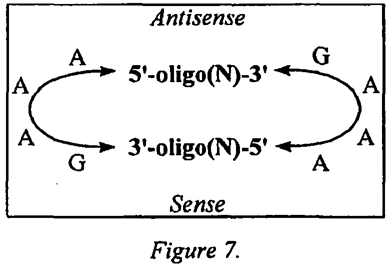

In examples in which loops are at one or both ends of the construct, the oligonucleotide can be divided by having a "nick" which is two non-linked nucleomonomers at any point along the sense or antisense strand, but preferably along the sense strand. Preferably, the nick is at least four bases from the nearest end of the duplexed region (to provide enough thermodynamic stability).

In another example, a construct of the invention can take the form: 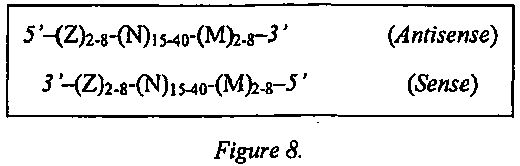 where the Ns are complementary nucleomonomers in oligonucleotide strands of equal length (*e.g*., between 12-40 nucleomonomers in length); Zs are nucleomonomers in complementary oligonucleotide strands of between about 2 and about 8 nucleomonomers in length and which comprise a sequence which can optionally correspond to the target sequence; and where Ms are nucleomonomers in complementary oligonucleotide strands of between about 2 and about 8 nucleomonomers in length and which can optionally correspond to the target sequence.

Preferably, the Zs and Ms are nucleomonomers selected from the group consisting of Cs and Gs to make the end of the duplex more thermodynamically stable. Ends of duplexes can become single stranded transiently, and since duplex RNA is more stable than single-stranded RNA, the enhanced stability of the duplex on the ends will result in higher nuclease stability.

An examplary sequence for Z or M in the antisense strand is from 2-8 nucleomonomers in length or preferably from 3-4 nucleomonomers in length, *e.g*., (from 5' to 3') CC, GG, CG, GC, CCC, GGG, CGG, GCC, GCG, CGC, CGGG, GCCC, CCCC, GGGG, GCGC, CGCG, GGGC, CCCG, CGGG, GCCC, GGCC, or CCGG. The complementary strand would have the corresponding complementary sequence.

In still another example, a construct of the invention has the form: 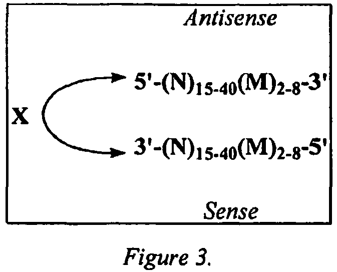 where Ns are nucleomonomers in complementary oligonucleotide strands (*i.e*., the top N strand is complementary to the bottom N strand) of equal length (*e.g*., from between about 12 to about 40 nucleomonomers in length) and X is selected from the group consisting of nothing (*i.e*., leaving blunt ends with no loop or overhang); 1-20 nucleotides of 5' overhang; 1-20 nucleotides of 3' overhang; a GAAA loop (tetra-loop); and a loop consisting of from about 4 to about 20 nucleomonomers (where the nucleomonomers are all either Gs or A's) and where Ms are nucleomonomers in complementary oligonucleotide strands of between about 2 and about 8 nucleomonomers in length (which can optionally correspond to the target sequence). Ms may be nucleomonomers selected from the group consisting of contain Cs and Gs.

An example sequence for M in the antisense strand is from 2-8 nucleomonomers in length or preferably from 3-4 nucleomonomers in length, *e.g.*, (from 5' to 3') CC, GG, CG, GC, CCC, GGG, CGG, GCC, GCG, CGC, CGGG, GCCC, CCCC, GGGG, GCGC, CGCG, GGGC, CCCG, CGGG, GCCC, GGCC, or CCGG and the corresponding complement on the opposite strand.

The construct may take the form: 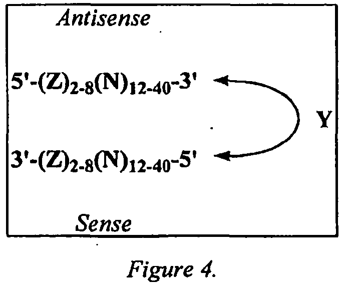 where Ns are nucleomonomers in complementary oligonucleotide strands of equal length (*e.g*., from between about 12 to about 40 nucleomonomers in length) and Y is selected from the group consisting of nothing (*i.e*., leaving blunt ends with no loop or overhang; 1-20 nucleotides of 5' overhang; 1-20 nucleotides of 3' overhang; a GAAA loop (tetra-loop); and a loop consisting of a sequence of from about 4 to about 20 nucleomonomers (where the nucleomonomers are all either Gs or A's) and where Zs are nucleomonomers in complementary oligonucleotide strands of between about 2 and about 8 nucleomonomers in length and which comprise a sequence which can optionally correspond to the target sequence. The Zs may be nucleomonomers selected from the group consisting of Cs and Gs to make the end of the duplex more stable.

An examplary sequence for Z in the antisense strand is from 2-8 nucleomonomers in length or preferably from 3-4 nucleomonomers in length, *e.g*., (from 5' to 3') CC, GG, CG, GC, CCC, GGG, CGG, GCC, GCG, CGC, CGGG, GCCC, CCCC, GGGG, GCGC, CGCG, GGGC, CCCG, CGGG, GCCC, GGCC or CCGG (and the corresponding complement on the opposite strand). For example, in the following structure, GGCC on the end (and its complement) confers additional stability:

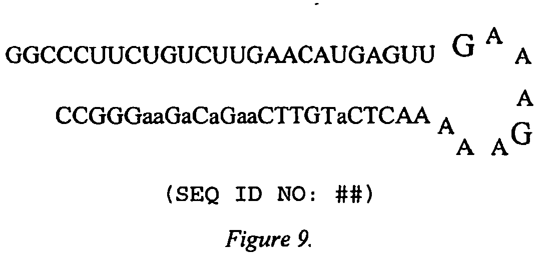

The description also relates to a double-stranded oligonucleotide composition having the following structure: 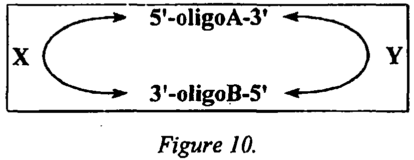 wherein (1) oligoA is an oligonucleotide of a number of nucleomonomers; (2) oligoB is an oligonucleotide that has the same number of nucleomonomers as oligoA and that is complementary to oligoA; (3)either oligoA or oligoB corresponds to a target gene sequence.

In this structure, X may be selected from (a) nothing; (b) an oligonucleotide of about 1 to about 20 nucleotides covalently bonded to the 5' end of oligoA and constituting a 5' overhang; (c) an oligonucleotide of about 1 to about 20 nucleotides covalently bonded to the 3' end of oligoB and constituting a 3' overhang; (d) and an oligonucleotide of about 4 to about 20 nucleomonomers covalently bonded to the 3' end of oligoB and the 5' end of oligoA and constituting a loop structure, where the nucleomonomers are selected from the group consisting of G and A.

Similarly, Y may be selected from (a) nothing; (b) an oligonucleotide of about 1 to about 20 nucleotides covalently bonded to the 5' end of oligoB and constituting a 5' overhang; (c) an oligonucleotide of about 1 to about 20 nucleotides covalently bonded to the 3' end of oligoA and constituting a 3' overhang; (d) and an oligonucleotide of about 4 to about 20 nucleomonomers covalently bonded to the 3' end of oligoA and the 5' end of oligoB and constituting a loop structure, where the nucleomonomers are selected from the group consisting of G and A.

Similarly, the description includes a double-stranded oligonucleotide composition having the structure: 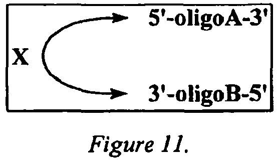 wherein (1) oligoA is 5'-(N))₁₅₋40-(M)₂₋₈-3' and oligoB is 5'-(N)₁₅₋40-(M)₂₋₈-3', wherein each of N and M is independently a nucleomonomer; (2) both of the sequences of Ns are complementary oligonucleotide strands of equal length having between about 15 and 40 nucleomonomers; (3) at least one of the sequences of Ns, optionally with some or all of the flanking Ms, corresponds to a target gene sequence. Both of the sequences of Ms are complementary oligonucleotide strands of between about 2 and about 8 nucleomonomers in length. The two M strands are optionally of the same length.

The group X indicated by the curved line is selected from (a) nothing; (b) an oligonucleotide of about 1 to about 20 nucleotides covalently bonded to the 5' end of oligoA and constituting a 5' overhang; (c) an oligonucleotide of about 1 to about 20 nucleotides covalently bonded to the 3' end of oligoB and constituting a 3' overhang; (d) and an oligonucleotide of about 4 to about 20 nucleomonomers covalently bonded to the 3' end of oligoB and the 5' end of oligoA and constituting a loop structure, where the nucleomonomers are selected from the group consisting of G and A.

Likewise, the descriptions pertains to a double-stranded oligonucleotide composition having the structure: 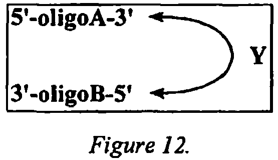 wherein (1) oligoA is 5'-(Z)₂₋₈-(N)₁₂₋₄₀-3' and oligoB is 5'-(Z)₂₋₈-(N)₁₂₋₄₀-3', wherein each of N and Z is independently a nucleomonomer; (2) both of the sequences of Ns are complementary oligonucleotide strands of equal length having between about 12 and 40 nucleomonomers; (3) at least one of the sequences ofNs, optionally with some or all of the flanking Zs, corresponds to a target gene sequence. Both of the sequences of Zs are complementary oligonucleotide strands of between about 2 and about 8 nucleomonomers in length. The two Z strands are optionally of the same length.

Here, Y is selected from (a) nothing; (b) an oligonucleotide of about 1 to about 20 nucleotides covalently bonded to the 5' end of oligoB and constituting a 5' overhang; (c) an oligonucleotide of about 1 to about 20 nucleotides covalently bonded to the 3' end of oligoA and constituting a 3' overhang; (d) and an oligonucleotide of about 4 to about 20 nucleomonomers covalently bonded to the 3' end of oligoA and the 5' end of oligoB and constituting a loop structure, where the nucleomonomers are selected from the group consisting of G and A.

In one embodiment the double-stranded nucleic acid molecule to be used in the method of the invention is between 15 and 30 nucleotides in length. In one embodiment, a double-stranded nucleic acid molecule 25, 27 or 21 in length.

The double-stranded nucleic acid molecule used in the invention is comprised of two separate nucleic acid molecules, the length of one of the individual nucleic acid molecules is between 18 and 30 nucleotides in length.

The double-stranded molecules used in the invention comprise a first nucleotide sequence which is antisense to at least part of the target gene and a second nucleotide sequence which is complementary to the first nucleotide sequence; *i.e*., is sense to at least part of the target gene. In one example, the second nucleotide sequence of the double-stranded molecule comprises a nucleotide sequence which is at least about 100 % complementary to the antisense molecule.

In another example, the second nucleotide sequence of the double-stranded molecule comprises a nucleotide sequence which is at least about 95 % complementary to the antisense molecule. In another embodiment, the second nucleotide sequence of the double-stranded molecule comprises a nucleotide sequence which is at least about 90 % complementary to the antisense molecule. In another example, the second nucleotide sequence of the double-stranded molecule comprises a nucleotide sequence which is at least about 80 % complementary to the antisense molecule. In another example, the second nucleotide sequence of the double-stranded molecule comprises a nucleotide sequence which is at least about 60 % complementary to the antisense molecule. In another example the second nucleotide sequence of the double-stranded molecule comprises a nucleotide sequence which is at least about 100 % complementary to the antisense molecule.

To determine the percent identity of two nucleic acid sequences, the sequences are aligned for optimal comparison purposes (*e.g*., gaps can be introduced in one or both of a first and a second amino acid or nucleic acid sequence for optimal alignment and non-identical sequences can be disregarded for comparison purposes). When a position in the first sequence is occupied by the same nucleotide as the corresponding position in the second sequence, then the molecules are identical at that position. The percent identity between the two sequences is a function of the number of identical positions shared by the sequences, taking into account the number of gaps, and the length of each gap, which need to be introduced for optimal alignment of the two sequences. The percent complementarity can be determined analogously; when a position in one sequence occupied by a nucleotide that is complementary to the nucleotide in the other sequence, then the molecules are complementary at that position.

The comparison of sequences and determination of percent identity between two sequences can be accomplished using a mathematical algorithm. In a example the percent identity between two nucleotide sequences is determined using *e.g*., the GAP program in the GCG software package, using a NWSgapdna. CMP matrix and a gap weight of 40, 50, 60, 70, or 80 and a length weight of 1, 2, 3, 4, 5, or 6. In another example, the percent identity between two nucleotide sequences is determined using the algorithm of E. Meyers and W. Miller (Comput. Appl. Biosci. 4:11-17 (1988)) which has been incorporated into the ALIGN program (version 2.0), using a PAM120 weight residue table, a gap length penalty of 12 and a gap penalty of 4.

The nucleic acid sequences of the present invention can further be used as a "query sequence" to perform alignments against sequences in public databases, Such searches can be performed using the NBLAST and XBLAST programs (version 2.0) of Altschul et al. (1990) J. Mol. Biol. 215:403-10. BLAST nucleotide searches can be performed with the NBLAST program, score = 100, wordlength = 12. To obtain gapped alignments for comparison purposes, Gapped BLAST can be utilized as described in Altschul et al. (1997) Nucleic Acids Res. 25(17):3389-3402. When utilizing BLAST and Gapped BLAST programs, the default parameters of the respective programs (*e.g*., XBLAST and NBLAST) can be used. *See*, *e*.*g*., the NIH website.

In yet another example a first antisense sequence of the double-stranded molecule hybridizes to its complementary second sequence of the double-stranded molecule under stringent hybridization conditions. As used herein, the term "hybridizes under stringent conditions" is intended to describe conditions for hybridization and washing under which nucleotide sequences at least 60% complementary to each other typically remain hybridized to each other. Preferably, the conditions are such that sequences at least about 70%, more preferably at least about 80%, even more preferably at least about 85% or 90% complementary to each other typically remain hybridized to each other.

Such stringent conditions are known to those skilled in the art and can be found in Current Protocols in Molecular Biology, John Wiley & Sons, N.Y. (1989),6.3.1-6.3.6. A preferred, non-limiting example of stringent hybridization conditions are hybridization in 6X sodium chloride/sodium citrate (SSC) at about 45°C, followed by one or more washes in 0.2 X SSC, 0.1% SDS at 50°C, preferably at 55°C, more preferably at 60°C, and even more preferably at 65°C. Ranges intermediate to the above-recited values, *e.g*., at 60-65°C or at 55-60°C are also intended to be encompassed by the present invention. Alternatively, formamide can be included in the hybridization solution, using methods and conditions also known in the art.

One of the sequences (or molecules) of the double-stranded oligonucleotide is antisense to the target gene. As used herein, the term "antisense sequence" includes nucleotide sequences which bind to the "sense" strand of the nucleotide sequence of the target gene (*e.g*., polynucleotides such as DNA, mRNA (including pre-mRNA)) molecules. When the antisense sequences bind to nucleic acid molecules, they can bind to any region of a nucleic acid molecule, including *e.g*., introns, exons, 5', or 3' untranslated regions. Antisense sequences that work by binding to a target and activating RNase H preferably bind within an intron, an exon, the 5' untranslated region, or the 3' untranslated region of a nucleic acid target molecule.

Preferably, the oligonucleotide compositions do not activate the interferon pathway, *e.g*., as evidenced by the lack of induction of the double-stranded RNA, interferon-inducible protein kinase, PKR.

In one example, modifications are made to a double-stranded RNA molecule which would normally activate the interferon pathway such that the interferon pathway is not activated. For example, the interferon pathway is activated by double-stranded unmodified RNA. The cellular recognition of double-stranded RNA is highly specific and modifying one or woth of the strands of a double-stranded duplex enable the double-stranded RNA molecule to evade the double-stranded RNA recognition machinery of the cell but would still allow for the activation of the RNAi pathway.

The ability of a double-stranded oligonucleotide to activate interferon could be assessed by testing for expression of the double-stranded RNA, Interferon-Inducible Protein Kinase, PKR using techniques known in the art and also testing for the ability of the double-stranded molecule to effect target gene inhibition.

Without being limited to any particular mechanism of action, an antisense sequence used in a double-stranded oligonucleotide composition that can specifically hybridize with a nucleotide sequence within the target gene (*i.e*., can be complementary to a nucleotide sequence within the target gene) may achieve its affects based on, *e.g*.,: (1) binding to target mRNA and stericly blocking the ribosome complex from translating the mRNA; (2) binding to target mRNA and triggering mRNA cleavage by RNase H; (3) binding to double-stranded DNA in the nucleus and forming a triple helix; (4) hybridizing to open DNA loops created by RNA polymerase; (5) interfering with mRNA splicing; (6) interfering with transport of mRNA from the nucleus to the cytoplasm; or (7) interfering with translation through inhibition of the binding of initiation factors or assembly of ribosomal subunits (*i.e*., at the start codon).

In one example, an antisense sequence of the double-stranded oligonucleotides of the invention is complementary to a target nucleic acid sequence over at least about 80% of the length of the antisense sequence. In another example the antisense sequence of the double-stranded oligonucleotide is complementary to a target nucleic acid sequence over at least about 90-95 % of the length of the antisense sequence. In another example, the antisense sequence of the double-stranded oligonucleotide is complementary to a target nucleic acid sequence over the entire length of the antisense sequence.

In yet another example, an antisense sequence of the double-stranded oligonucleotide hybridizes to at least a portion of the target gene under stringent hybridization conditions.

In one example, antisense sequences are substantially complementary to a target nucleic acid sequence. In one example, an antisense RNA molecule comprises a nucleotide sequence which is at least about 100 % complementary to a portion of the target gene. In another example, an antisense RNA molecule comprises a nucleotide sequence which is at least about 90 % complementary to a portion of the target gene. In another example, an antisense RNA molecule comprises a nucleotide sequence which is at least about 80 % complementary to a portion of the target gene. In another example, an antisense RNA molecule comprises a nucleotide sequence which is at least about 60 % complementary to a portion of the target gene. In another example, an antisense RNA molecule comprises a nucleotide sequence which is at least about 100 % complementary to a portion of the target gene. Preferably, no loops greater than about 8 nucleotides are formed by areas of non-complementarity between the oligonucleotide and the target.

In one example, an antisense nucleotide sequence of the invention is complementary to a target nucleic acid sequence over at least about 80% of the length of the antisense sequence. In another example, an antisense sequence is complementary to a target nucleic acid sequence over at least about 90-95 % of the length of the antisense sequence. In another example, an antisense sequence is complementary to a target nucleic acid sequence over the entire length of the antisense sequence.

The antisense sequences used in an oligonucleotide composition may be of any type, *e.g*., including morpholino oligonucleotides, RNase H activating oligonucleotides, or ribozymes.

In one example, a double-stranded oligonucleotide can comprise (*i.e*., be a duplex of) one nucleic acid molecule which is DNA and one nucleic acid molecule which is RNA.

Antisense sequences be "chimeric oligonucleotides" which comprise an RNA-like and a DNA-like region. The language "RNase H activating region" includes a region of an oligonucleotide, *e.g*., a chimeric oligonucleotide, that is capable of recruiting RNase H to cleave the target RNA strand to which the oligonucleotide binds. Typically, the RNase activating region contains a minimal core (of at least about 3-5, typically between about 3-12, more typically, between about 5-12, and more preferably between about 5-10 contiguous nucleomonomers) of DNA or DNA-like nucleomonomers. (*See*, *e.g*., US patent 5,849,902). Preferably, the RNase H activating region comprises about nine contiguous deoxyribose containing nucleomonomers.

In one example, the contiguous nucleomonomers are linked by a substitute linkage, *e.g*., a phosphorothioate linkage. In one example, an antisense sequence of the invention is unstable, *i.e*., is degraded in a cell, in the absence of the second strand (or self complementary sequence) which forms a double-stranded oligonucleotide of the invention. For example, a chimeric antisense sequence comprises unmodified DNA nucleomonomers in the gap rather than phosphorothioate DNA.

The language "non-activating region" includes a region of an antisense sequence, *e.g*., a chimeric oligonucleotide, that does not recruit or activate RNase H. Preferably, a non-activating region does not comprise phosphorothioate DNA. The oligonucleotides comprise at least one non-activating region. In one example, the non-activating region can be stabilized against nucleases or can provide specificity for the target by being complementary to the target and forming hydrogen bonds with the target nucleic acid molecule, which is to be bound by the oligonucleotide.

Antisense sequences may include "morpholino oligonucleotides." Morpholino oligonucleotides are non-ionic and function by an RNase H-independent mechanism. Each of the 4 genetic bases (Adenine, Cytosine, Guanine, and Thymine/Uracil) of the morpholino oligonucleotides is linked to a 6-membered morpholine ring. Morpholino oligonucleotides are made by joining the 4 different subunit types by, *e.g*., non-ionic phosphorodiamidate inter-subunit linkages. An example of a 2 subunit morpholino oligonucleotide is shown below.

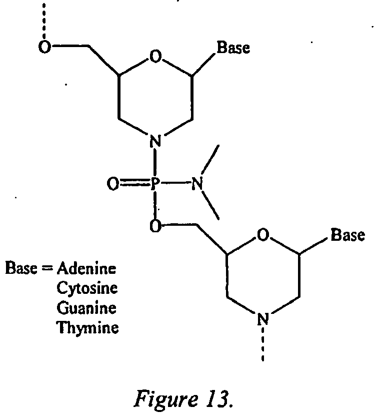

Morpholino oligonucleotides have many advantages including: complete resistance to nucleases (Antisense & Nuc. Acid Drug Dev. 1996. 6:267); predictable targeting (Biochemica Biophysica Acta. 1999. 1489:141); reliable activity in cells (Antisense & Nuc. Acid Drug Dev. 1997. 7:63); excellent sequence specificity (Antisense & Nuc. Acid Drug Dev. 1997. 7:151); minimal non-antisense activity (Biochemica Biophysica Acta. 1999. 1489:141); and simple osmotic or scrape delivery (Antisense & Nuc. Acid Drug Dev. 1997. 7:291). Morpholino oligonucleotides are also preferred because of their non-toxicity at high doses. A discussion of the preparation of morpholino oligonucleotides can be found in Antisense & Nuc. Acid Drug Dev. 1997. 7:187.

### Uptake Of Oligonucleotides By Cells

Oligonucleotides and oligonucleotide compositions are contacted with (*i.e*., brought into contact with, also referred to herein as administered or delivered to) and taken up by one or more cells or a cell lysate. The term "cells" includes prokaryotic and eukaryotic cells, preferably vertebrate cells, and, more preferably, mammalian cells. In one example , the oligonucleotide compositions are contacted with human cells.

Oligonucleotide compositions can be contacted with cells *in vitro, e.g*., in a test tube or culture dish, (and may or may not be introduced into a subject) or *in vivo*, *e*.*g*., in a subject such as a mamalian subject. Oligonucleotides are taken up by cells at a slow rate by endocytosis, but endocytosed oligonucleotides are generally sequestered and not available, *e.g*., for hybridization to a target nucleic acid molecule. In one embodiment, cellular uptake can be facilitated by electroporation . However, these procedures are only useful for *in vitro* or *ex vivo* embodiments, are not convenient and, in some cases, are associated with cell toxicity.

In another example, delivery of oligonucleotides into cells can be enhanced by suitable art recognized methods including calcium phosphate, DMSO, glycerol or dextran, electroporation, or by transfection, *e.g*., using cationic, anionic, or neutral lipid compositions or liposomes using methods known in the art (see *e.g*., WO 90/14074; WO 91/16024; WO 91/17424; U.S.Patent No. 4,897,355; Bergan et al. 1993. Nucleic Acids Research. 21:3567). Enhanced delivery of oligonucleotides can also be mediated by the use of vectors (See *e.g*., Shi, Y. 2003. Trends Genet 2003 Jan 19:9; Reichhart JM et al. Genesis. 2002. 34(1-2):160-4, Yu et al. 2002. Proc Natl Acad Sci U S A 99:6047; Sui et al. 2002. Proc Natl Acad Sci U S A 99:5515) viruses, polyamine or polycation conjugates using compounds such as polylysine, protamine, or N1, N12-bis (ethyl) spermine (*see*, *e.g*., Bartzatt, R. et al. 1989. Biotechnol. Appl. Biochem. 11:133; Wagner E. et al. 1992. Proc. Natl. Acad. Sci. 88:4255)

### Conjugating Agents

Conjugating agents bind to the oligonucleotide in a covalent manner. In one example, oligonucleotides can be derivitized or chemically modified by binding to a conjugating agent to facilitate cellular uptake. For example, covalent linkage of a cholesterol moiety to an oligonucleotide can improve cellular uptake by 5- to 10- fold which in turn improves DNA binding by about 10- fold (Boutorin et al., 1989, FEBS Letters 254:129-132). Conjugation of octyl, dodecyl, and octadecyl residues enhances cellular uptake by 3-, 4-, and 10- fold as compared to unmodified oligonucleotides (Vlassov et al., 1994, Biochimica et Biophysica Acta 1197:95-108). Similarly, derivatization of oligonucleotides with poly-L-lysine can aid oligonucleotide uptake by cells (Schell, 1974, Biochem. Biophys. Acta 340:323, and Lemaitre et al., 1987, Proc. Natl. Acad. Sci. USA 84:648).

Certain protein carriers can also facilitate cellular uptake of oligonucleotides, including, for example, serum albumin, nuclear proteins possessing signals for transport to the nucleus, and viral or bacterial proteins capable of cell membrane penetration. Therefore, protein carriers are useful when associated with or linked to the oligonucleotides. Accordingly, the present description provides for derivatization of oligonucleotides with groups capable of facilitating cellular uptake, including hydrocarbons and non-polar groups, cholesterol, long chain alcohols (*i.e*., hexanol), poly-L-lysine and proteins, as well as other aryl or steroid groups and polycations having analogous beneficial effects, such as phenyl or naphthyl groups, quinoline, anthracene or phenanthracene groups, fatty acids, fatty alcohols and sesquiterpenes, diterpenes, and steroids. A major advantage of using conjugating agents is to increase the initial membrane interaction that leads to a greater cellular accumulation of oligonucleotides.

### Encapsulating Agents

Encapsulating agents entrap oligonucleotides within vesicles. In another example , an oligonucleotide may be associated with a carrier or vehicle, *e.g*., liposomes or micelles, although other carriers could be used, as would be appreciated by one skilled in the art. Liposomes are vesicles made of a lipid bilayer having a structure similar to biological membranes. Such carriers are used to facilitate the cellular uptake or targeting of the oligonucleotide, or improve the oligonucleotide's pharmacokinetic or toxicologic properties.

For example, the oligonucleotides may also be administered encapsulated in liposomes, pharmaceutical compositions wherein the active ingredient is contained either dispersed or variously present in corpuscles consisting of aqueous concentric layers adherent to lipidic layers. The oligonucleotides, depending upon solubility, may be present both in the aqueous layer and in the lipidic layer, or in what is generally termed a liposomic suspension. The hydrophobic layer, generally but not exclusively, comprises phopholipids such as lecithin and sphingomyelin, steroids such as cholesterol, more or less ionic surfactants such as diacetylphosphate, stearylamine, or phosphatidic acid, or other materials of a hydrophobic nature. The diameters of the liposomes generally range from about 15 nm to about 5 microns.

The use of liposomes as drug delivery vehicles offers several advantages. Liposomes increase intracellular stability, increase uptake efficiency and improve biological activity. Liposomes are hollow spherical vesicles composed of lipids arranged in a similar fashion as those lipids which make up the cell membrane. They have an internal aqueous space for entrapping water soluble compounds and range in size from 0.05 to several microns in diameter. Several studies have shown that liposomes can deliver nucleic acids to cells and that the nucleic acids remain biologically active. For example, a liposome delivery vehicle originally designed as a research tool, such as Lipofectin, can deliver intact nucleic acid molecules to cells.

Specific advantages of using liposomes include the following: they are non-toxic and biodegradable in composition; they display long circulation half-lives; and recognition molecules can be readily attached to their surface for targeting to tissues. Finally, cost-effective manufacture of liposome-based pharmaceuticals, either in a liquid suspension or lyophilized product, has demonstrated the viability of this technology as an acceptable drug delivery system.

### Complexing Agents

Complexing agents bind to the oligonucleotides by a strong but noncovalent attraction (*e.g*., an electrostatic, van der Waals, pi-stacking, etc. interaction). In one example, oligonucleotides can be complexed with a complexing agent to increase cellular uptake of oligonucleotides. An example of a complexing agent includes cationic lipids. Cationic lipids can be used to deliver oligonucleotides to cells.

The term "cationic lipid" includes lipids and synthetic lipids having both polar and non-polar domains and which are capable of being positively charged at or around physiological pH and which bind to polyanions, such as nucleic acids, and facilitate the delivery of nucleic acids into cells. In general cationic lipids include saturated and unsaturated alkyl and alicyclic ethers and esters of amines, amides, or derivatives thereof. Straight-chain and branched alkyl and alkenyl groups of cationic lipids can contain, *e.g*., from 1 to about 25 carbon atoms. Preferred straight chain or branched alkyl or alkene groups have six or more carbon atoms. Alicyclic groups include cholesterol and other steroid groups. Cationic lipids can be prepared with a variety of counterions (anions) including, *e.g*., Cl⁻, Br⁻, I⁻, F⁻, acetate, trifluoroacetate, sulfate, nitrite, and nitrate.

Examples of cationic lipids include polyethylenimine, polyamidoamine (PAMAM) starburst dendrimers, Lipofectin (a combination of DOTMA and DOPE), Lipofectase, Lipofectamine, DOPE, Cytofectin (Gilead Sciences, Foster City, CA), and Eufectins (JBL, San Luis Obispo, CA). Exemplary cationic liposomes can be made from N-[1-(2,3-dioleoloxy)-propyl]-N,N,N-trimethylammonium chloride (DOTMA), N-[1-(2,3-dioleoloxy)-propyl]-N,N,N-trimethylammonium methylsulfate (DOTAP), 3β-[N-N',N'-dimethylaminoethane)carbamoyl]cholesterol (DC-Chol), 2,3,-dioleyloxy-N-[2(sperminecarboxamido)ethyl]-N,N-dimethyl-1-propanaminium trifluoroacetate (DO SPA), 1,2-dimyristyloxypropyl-3-dimethyl-hydroxyethyl ammonium bromide; and dimethyldioctadecylammonium bromide (DDAB). The cationic lipid N-(1-(2,3-dioleyloxy)propyl)-N,N,N-trimethylammonium chloride (DOTMA), for example, was found to increase 1000-fold the antisense effect of a phosophorothioate oligonucleotide. (Vlassov et al., 1994, Biochimica et Biophysica Acta 1197:95-108). Oligonucleotides can also be complexed with, *e.g*., poly (L-lysine) or avidin and lipids may, or may not, be included in this mixture, *e.g*., steryl-poly (L-lysine).

Cationic lipids have been used in the art to deliver oligonucleotides to cells (*see*, *e.g*., U.S. 5,855,910; 5,851,548; 5,830,430; 5,780,053; 5,767,099; Lewis et al. 1996. Proc. Natl. Acad. Sci. USA 93:3176; Hope et al. 1998. Molecular Membrane Biology 15:1). Other lipid compositions which can be used to facilitate uptake of the instant oligonucleotides can be used in connection with the claimed methods. In addition to those listed supra, other lipid compositions are also known in the art and include, *e.g*., those taught in U.S. patent 4,235,871; U.S. patents 4,501,728; 4,837,028; 4,737,323.

In one example lipid compositions can further comprise agents, *e.g*., viral proteins to enhance lipid-mediated transfections of oligonucleotides (Kamata, et al., 1994. Nucl. Acids. Res. 22:536). In another example, oligonucleotides are contacted with cells as part of a composition comprising an oligonucleotide, a peptide, and a lipid as taught, *e.g*., in U.S. patent 5,736,392. Improved lipids have also been described which are serum resistant (Lewis, et al., 1996. Proc. Natl. Acad. Sci. 93:3176). Cationic lipids and other complexing agents act to increase the number of oligonucleotides carried into the cell through endocytosis.

In another example N-substituted glycine oligonucleotides (peptoids) can be used to optimize uptake of oligonucleotides. Peptoids have been used to create cationic lipid-like compounds for transfection (Murphy, et al., 1998. Proc. Natl. Acad. Sci. 95:1517). Peptoids can be synthesized using standard methods (*e.g*., Zuckermann, R. N., et al. 1992, J. Am. Chem. Soc. 114:10646; Zuckermann, R.N., et al. 1992. Int. J. Peptide Protein Res. 40:497). Combinations of cationic lipids and peptoids, liptoids, can also be used to optimize uptake of the subject oligonucleotides (Hunag, et al., 1998. Chemistry and Biology. 5:345). Liptoids can be synthesized by elaborating peptoid oligonucleotides and coupling the amino terminal submonomer to a lipid via its amino group (Hunag, et al., 1998. Chemistry and Biology. 5:345).

It is known in the art that positively charged amino acids can be used for creating highly active cation lipids (Lewis et al. 1996. Proc. Natl. Acad. Sci. U.S.A. 93:3176). In one example, a composition for delivering oligonucleotides of the invention comprises a number of arginine, lysine, histadine or ornithine residues linked to a lipophilic moiety (see *e.g.,* U.S. patent 5,777,153).

In another, a composition for delivering oligonucleotides comprises a peptide having from between about one to about four basic residues. These basic residues can be located, *e.g*., on the amino terminal, C-terminal, or internal region of the peptide. Families of amino acid residues having similar side chains have been defined in the art. These families include amino acids with basic side chains (*e.g*., lysine, arginine, histidine), acidic side chains (*e.g*., aspartic acid, glutamic acid), uncharged polar side chains (*e.g*., glycine (can also be considered non-polar), asparagine, glutamine, serine, threonine, tyrosine, cysteine), nonpolar side chains (*e.g*., alanine, valine, leucine, isoleucine, proline, phenylalanine, methionine, tryptophan), beta-branched side chains (*e.g*., threonine, valine, isoleucine) and aromatic side chains (*e.g*., tyrosine, phenylalanine, tryptophan, histidine). Apart from the basic amino acids, a majority or all of the other residues of the peptide can be selected from the non-basic amino acids, *e.g*., amino acids other than lysine, arginine, or histidine. Preferably a preponderance of neutral amino acids with long neutral side chains are used. For example, a peptide such as (N-term) His-Ile-Trp-Leu-Ile-Tyr-Leu-Trp-Ile-Val-(C-term) (SEQ ID NO: ##) could be used. In one example such a composition can be mixed with the fusogenic lipid DOPE as is well known in the art.

In one example, the cells to be contacted with an oligonucleotide composition are contacted with a mixture comprising the oligonucleotide and a mixture comprising a lipid, *e.g*., one of the lipids or lipid compositions described supra for between about 12 h to about 24 h. In another example, the cells to be contacted with an oligonucleotide composition are contacted with a mixture comprising the oligonucleotide and a mixture comprising a lipid, *e.g*., one of the lipids or lipid compositions described supra for between about 1 and about five days. In one example, the cells are contacted with a mixture comprising a lipid and the oligonucleotides for between about three days to as long as about 30 days. In another example, a mixture comprising a lipid is left in contact with the cells for at least about five to about 20 days. In another example, a mixture comprising a lipid is left in contact with the cells for at least about seven to about 15 days.

For example, an oligonucleotide composition can be contacted with cells in the presence of a lipid such as cytofectin CS or GSV(available from Glen Research; Sterling, VA), GS3815, GS2888 for prolonged incubation periods as described herein.

In one example the incubation of the cells with the mixture comprising a lipid and an oligonucleotide composition does not reduce the viability of the cells. Preferably, after the transfection period the cells are substantially viable. In one example, after transfection, the cells are between at least about 70 and at least about 100 percent viable. In another example, the cells are between at least about 80 and at least about 95% viable. In yet another example, the cells are between at least about 85% and at least about 90% viable.

Oligonucleotides may be modified by attaching a peptide sequence that transports the oligonucleotide into a cell, referred to herein as a "transporting peptide." In one example, the composition includes an oligonucleotide which is complementary to a target nucleic acid molecule encoding the protein, and a covalently attached transporting peptide.

The language "transporting peptide" includes an amino acid sequence that facilitates the transport of an oligonucleotide into a cell. Exemplary peptides which facilitate the transport of the moieties to which they are linked into cells are known in the art, and include, *e.g*., HIV TAT transcription factor, lactoferrin, Herpes VP22 protein, and fibroblast growth factor 2 (Pooga et al. 1998. Nature Biotechnology 16:857; and Derossi et al. 1998. Trends in Cell Biology. 8:84; Elliott and O'Hare. 1997. Cell 88:223).

For example, the transporting peptide comprises an amino acid sequence derived from the antennapedia protein. Preferably, the peptide comprises amino acids 43-58 of the antennapedia protein (Arg-Gln-Ile-Lys-Ile-Trp-Phe-Gln-Asn-Arg-Arg-Met-Lys-Trp-Lys-Lys) (SEQ ID NO: ##) or a portion or variant thereof that facilitates transport of an oligonucleotide into a cell (*see*, *e.g*., WO 91/1898; Derossi et al. 1998. Trends Cell Biol. 8:84). Exemplary variants are shown in Derossi *et al.,* supra.

In one example, the transporting peptide comprises an amino acid sequence derived from the transportan, galanin (1-12)-Lys-mastoparan (1-14) amide, protein. (Pooga et al. 1998. Nature Biotechnology 16:857). Preferably, the peptide comprises the amino acids of the transportan protein shown in the sequence GWTLNSAGYLLGKINLKAL-AALAKKIL (SEQ ID NO: ##) or a portion or variant thereof that facilitates transport of an oligonucleotide into a cell.

In one example, the transporting peptide comprises an amino acid sequence derived from the HIV TAT protein. Preferably, the peptide comprises amino acids 37-72 of the HIV TAT protein, *e.g*., shown in the sequence C(Acm)FITKALGISYGRKKRRQRRR-PPQC (SEQ ID NO: ##) (TAT 37-60; where C(Acm) is Cys-acetamidomethyl) or a portion or variant thereof, *e.g*., C(Acm)GRKKRRQRRRPPQC (SEQ ID NO: ##) (TAT 48-40) or C(Acm)LGISYGRKKRRQRRPPQC (SEQ ID NO: ##) (TAT 43-60) that facilitates transport of an oligonucleotide into a cell (Vives et al. 1997. J. Biol. Chem. 272:16010). In another example the peptide (G)CFITKALGISYGRKKRRQR-RRPPQGSQTHQVSLSKQ (SEQ ID NO: ##) can be used.

Portions or variants of transporting peptides can be readily tested to determine whether they are equivalent to these peptide portions by comparing their activity to the activity of the native peptide, *e.g*., their ability to transport fluorescently-labeled oligonucleotides to cells. Fragments or variants that retain the ability of the native transporting peptide to transport an oligonucleotide into a cell are functionally equivalent and can be substituted for the native peptides.

Oligonucleotides can be attached to the transporting peptide using known techniques, *e.g.,* (Prochiantz, A. 1996. Curr. Opin. Neurobiol. 6:629; Derossi et al. 1998. Trends Cell Biol. 8:84; Troy et al. 1996. J. Neurosci. 16:253), Vives et al. 1997. J. Biol. Chem. 272:16010). For example, oligonucleotides bearing an activated thiol group are linked via that thiol group to a cysteine present in a transport peptide (*e.g*., to the cysteine present in the β turn between the second and the third helix of the antennapedia homeodomain as taught, *e.g.*, in Derossi et al. 1998. Trends Cell Biol. 8:84; Prochiantz. 1996. Current Opinion in Neurobiol. 6:629; Allinquant et al. 1995. J. Cell Biol. 128:919). In another embodiment, a Boc-Cys-(Npys)OH group can be coupled to the transport peptide as the last (N-terminal) amino acid and an oligonucleotide bearing an SH group can be coupled to the peptide (Troy et al. 1996. J. Neurosci. 16:253).

In one example, a linking group can be attached to a nucleomonomer and the transporting peptide can be covalently attached to the linker. In example, a linker can function as both an attachment site for a transporting peptide and can provide stability against nucleases. Examples of suitable linkers include substituted or unsubstituted C₁-C₂₀ alkyl chains, C₂-C₂₀ alkenyl chains, C₂-C₂₀ alkynyl chains, peptides, and heteroatoms (*e.g*., S, O, NH, etc.). Other exemplary linkers include bifunctional crosslinking agents such as sulfosuccinimidyl-4-(maleimidophenyl)-butyrate (SMPB) (*see*, *e.g*., Smith et al. Biochem J 1991. 276: 417-2).

In one example, oligonucleotides are synthesized as molecular conjugates which utilize receptor-mediated endocytotic mechanisms for delivering genes into cells (*see*, *e.g*., Bunnell et al. 1992. Somatic Cell and Molecular Genetics. 18:559 and the references cited therein).

### Targeting Agents

The delivery of oligonucleotides can also be improved by targeting the oligonucleotides to a cellular receptor. The targeting moieties can be conjugated to the oligonucleotides or attached to a carrier group (*i.e*., poly(L-lysine) or liposomes) linked to the oligonucleotides. This method is well suited to cells that display specific receptor-mediated endocytosis.

For instance, oligonucleotide conjugates to 6-phosphomannosylated proteins are internalized 20-fold more efficiently by cells expressing mannose 6-phosphate specific receptors than free oligonucleotides. The oligonucleotides may also be coupled to a ligand for a cellular receptor using a biodegradable linker. In another example, the delivery construct is mannosylated streptavidin which forms a tight complex with biotinylated oligonucleotides. Mannosylated streptavidin was found to increase 20-fold the internalization of biotinylated oligonucleotides. (Vlassov et al. 1994. Biochimica et Biophysica Acta 1197:95-108).

In addition specific ligands can be conjugated to the polylysine component of polylysine-based delivery systems. For example, transferrin-polylysine, adenovirus-polylysine, and influenza virus hemagglutinin HA-2 N-terminal fusogenic peptides-polylysine conjugates greatly enhance receptor-mediated DNA delivery in eucaryotic cells. Mannosylated glycoprotein conjugated to poly(L-lysine) in aveolar macrophages has been employed to enhance the cellular uptake of oligonucleotides. Liang et al. 1999. Pharmazie 54:559-566.

Because malignant cells have an increased need for essential nutrients such as folic acid and transferrin, these nutrients can be used to target oligonucleotides to cancerous cells. For example, when folic acid is linked to poly(L-lysine) enhanced oligonucleotide uptake is seen in promyelocytic leukaemia (HL-60) cells and human melanoma (M-14) cells. Ginobbi et al. 1997. Anticancer Res. 17:29. In another example, liposomes coated with maleylated bovine serum albumin, folic acid, or ferric protoporphyrin IX, show enhanced cellular uptake of oligonucleotides in murine macrophages, KB cells, and 2.2.15 human hepatoma cells. Liang et al. 1999. Pharmazie 54:559-566.

Liposomes naturally accumulate in the liver, spleen, and reticuloendothelial system (so-called, passive targeting). By coupling liposomes to various ligands such as antibodies are protein A, they can be actively targeted to specific cell populations. For example, protein A-bearing liposomes may be pretreated with H-2K specific antibodies which are targeted to the mouse major histocompatibility complex-encoded H-2K protein expressed on L cells. (Vlassov et al. 1994. Biochimica et Biophysica Acta 1197:95-108).

### Assays of Oligonucleotide Stability

Preferably, the double-stranded oligonucleotides are stabilized, *i.e*., substantially resistant to endonuclease and exonuclease degradation. An oligonucleotide is defined as being substantially resistant to nucleases when it is at least about 3-fold more resistant to attack by an endogenous cellular nuclease, and is highly nuclease resistant when it is at least about 6-fold more resistant than a corresponding, single-stranded oligonucleotide. This can be demonstrated by showing that the oligonucleotides are substantially resist nucleases using techniques which are known in the art.

One way in which substantial stability can be demonstrated is by showing that the oligonucleotides function when delivered to a cell, *e.g*., that they reduce transcription or translation of target nucleic acid molecules, *e.g*., by measuring protein levels or by measuring cleavage of mRNA. Assays which measure the stability of target RNA can be performed at about 24 hours post-transfection (*e.g*., using Northern blot techniques, RNase Protection Assays, or QC-PCR assays as known in the art). Alternatively, levels of the target protein can be measured. Preferably, in addition to testing the RNA or protein levels of interest, the RNA or protein levels of a control, non-targeted gene will be measured (*e.g*., actin, or preferably a control with sequence similarity to the target) as a specificity control. RNA or protein measurements can be made using any art-recognized technique. Preferably, measurements will be made beginning at about 16-24 hours post transfection. (M. Y. Chiang, et al. 1991. J Biol Chem. 266:18162-71; T. Fisher, et al. 1993. Nucleic Acids Research. 21 3857).

The ability of an oligonucleotide composition to inhibit protein synthesis can be measured using techniques which are known in the art, for example, by detecting an inhibition in gene transcription or protein synthesis. For example, Nuclease S1 mapping can be performed. In another example, Northern blot analysis can be used to measure the presence of RNA encoding a particular protein. For example, total RNA can be prepared over a cesium chloride cushion (*see*, *e.g*., Ausebel et al., 1987. Current Protocols in Molecular Biology (Greene & Wiley, New York)). Northern blots can then be made using the RNA and probed (*see*, *e.g*., Id.). In another example, the level of the specific mRNA produced by the target protein can be measured, *e.g*., using PCR. In yet another example, Western blots can be used to measure the amount of target protein present. In still another example, a phenotype influenced by the amount of the protein can be detected. Techniques for performing Western blots are well known in the art, *see*, *e.g*., Chen et al. J. Biol. Chem. 271:28259.

In another example, the promoter sequence of a target gene can be linked to a reporter gene and reporter gene transcription (*e.g*., as described in more detail below) can be monitored. Alternatively, oligonucleotide compositions that do not target a promoter can be identified by fusing a portion of the target nucleic acid molecule with a reporter gene so that the reporter gene is transcribed. By monitoring a change in the expression of the reporter gene in the presence of the oligonucleotide composition, it is possible to determine the effectiveness of the oligonucleotide composition in inhibiting the expression of the reporter gene. For example, an effective oligonucleotide composition will reduce the expression of the reporter gene.

A "reporter gene" is a nucleic acid that expresses a detectable gene product, which may be RNA or protein. Detection of mRNA expression may be accomplished by Northern blotting and detection of protein may be accomplished by staining with antibodies specific to the protein. Preferred reporter genes produce a readily detectable product. A reporter gene may be operably linked with a regulatory DNA sequence such that detection of the reporter gene product provides a measure of the transcriptional activity of the regulatory sequence. In some examples the gene product of the reporter gene is detected by an intrinsic activity associated with that product. For instance, the reporter gene may encode a gene product that, by enzymatic activity, gives rise to a detectable signal based on color, fluorescence, or luminescence. Examples of reporter genes include, but are not limited to, those coding for chloramphenicol acetyl transferase (CAT), luciferase, β-galactosidase, and alkaline phosphatase.

One skilled in the art would readily recognize numerous reporter genes suitable for use. These include, but are not limited to, chloramphenicol acetyltransferase (CAT), luciferase, human growth hormone (hGH), and beta-galactosidase. Examples of such reporter genes can be found in F. A. Ausubel et al., Eds., Current Protocols in Molecular Biology, John Wiley & Sons, New York, (1989). Any gene that encodes a detectable product, *e.g*., any product having detectable enzymatic activity or against which a specific antibody can be raised, can be used as a reporter gene in the present methods.

One reporter gene system is the firefly luciferase reporter system. (Gould, S. J., and Subramani, S. 1988. Anal. Biochem., 7:404-408). The luciferase assay is fast and sensitive. In this assay, a lysate of the test cell is prepared and combined with ATP and the substrate luciferin. The encoded enzyme luciferase catalyzes a rapid, ATP dependent oxidation of the substrate to generate a light-emitting product. The total light output is measured and is proportional to the amount of luciferase present over a wide range of enzyme concentrations.

CAT is another frequently used reporter gene system; a major advantage of this system is that it has been an extensively validated and is widely accepted as a measure of promoter activity. (Gorman C. M., Moffat, L. F., and Howard, B. H. 1982. Mol. Cell. Biol., 2:1044-1051). In this system, test cells are transfected with CAT expression vectors and incubated with the candidate substance within 2-3 days of the initial transfection. Thereafter, cell extracts are prepared. The extracts are incubated with acetyl CoA and radioactive chloramphenicol. Following the incubation, acetylated chloramphenicol is separated from nonacetylated form by thin layer chromatography. In this assay, the degree of acetylation reflects the CAT gene activity with the particular promoter.

Another suitable reporter gene system is based on immunologic detection of hGH. This system is also quick and easy to use. (Selden, R., Burke-Howie, K. Rowe, M. E., Goodman, H. M., and Moore, D. D. (1986), Mol. Cell, Biol., 6:3173-3179 ). The hGH system is advantageous in that the expressed hGH polypeptide is assayed in the media, rather than in a cell extract. Thus, this system does not require the destruction of the test cells. It will be appreciated that the principle of this reporter gene system is not limited to hGH but rather adapted for use with any polypeptide for which an antibody of acceptable specificity is available or can be prepared.

In one example, nuclease stability of a double-stranded oligonucleotide is measured and compared to a control, *e.g.*, an RNAi molecule typically used in the art (*e.g*., a duplex oligonucleotide of less than 25 nucleotides in length and comprising 2 nucleotide base overhangs) or an unmodified RNA duplex with blunt ends.

### Oligonucleotide Synthesis

Oligonucleotides can be synthesized by any method known in the art, *e.g*., using enzymatic synthesis and chemical synthesis. The oligonucleotides can be synthesized *in vitro* (*e.g*., using enzymatic synthesis and chemical synthesis) or *in vivo* (using recombinant DNA technology well known in the art).

In a preferred example, chemical synthesis is used. Chemical synthesis of linear oligonucleotides is well known in the art and can be achieved by solution or solid phase techniques. Preferably, synthesis is by solid phase methods. Oligonucleotides can be made by any of several different synthetic procedures including the phosphoramidite, phosphite triester, H-phosphonate, and phosphotriester methods, typically by automated synthesis methods.

Oligonucleotide synthesis protocols are well known in the art and can be found, *e.g*., in U.S. patent 5,830,653; WO 98/13526; Stec et al. 1984. J. Am. Chem. Soc. 106:6077; Stec et al. 1985. J. Org. Chem. 50:3908; Stec et al. J. Chromatog. 1985. 326:263; LaPlanche et al. 1986. Nuc. Acid Res. 1986. 14:9081; Fasman G. D., 1989. Practical Handbook of Biochemistry and Molecular Biology. 1989. CRC Press, Boca Raton, Fla.; Lamone. 1993. Biochem. Soc. Trans. 21:1; U.S. Patent 5,013,830; U.S. Patent 5,214,135; U.S. Patent 5,525,719; Kawasaki et al. 1993. J. Med. Chem. 36:831; WO 92/03568; U.S. Patent 5,276,019; U.S. Patent 5,264,423.

The synthesis method selected can depend on the length of the desired oligonucleotide and such choice is within the skill of the ordinary artisan. For example, the phosphoramidite and phosphite triester method can produce oligonucleotides having 175 or more nucleotides while the H-phosphonate method works well for oligonucleotides of less than 100 nucleotides. If modified bases are incorporated into the oligonucleotide, and particularly if modified phosphodiester linkages are used, then the synthetic procedures are altered as needed according to known procedures. In this regard, Uhlmann et al. (1990, Chemical Reviews 90:543-584) provide references and outline procedures for making oligonucleotides with modified bases and modified phosphodiester linkages. Other exemplary methods for making oligonucleotides are taught in Sonveaux. 1994. "Protecting Groups in Oligonucleotide Synthesis"; Agrawal. Methods in Molecular Biology 26:1. Exemplary synthesis methods are also taught in "Oligonucleotide Synthesis- A Practical Approach" (Gait, M.J. IRL Press at Oxford University Press. 1984). Moreover, linear oligonucleotides of defined sequence, including some sequences with modified nucleotides, are readily available from several commercial sources.

The oligonucleotides may be purified by polyacrylamide gel electrophoresis, or by any of a number of chromatographic methods, including gel chromatography and high pressure liquid chromatography. To confirm a nucleotide sequence, oligonucleotides may be subjected to DNA sequencing by any of the known procedures, including Maxam and Gilbert sequencing, Sanger sequencing, capillary electrophoresis sequencing the wandering spot sequencing procedure or by using selective chemical degradation of oligonucleotides bound to Hybond paper. Sequences of short oligonucleotides can also be analyzed by laser desorption mass spectroscopy or by fast atom bombardment (McNeal, et al., 1982, J. Am. Chem. Soc. 104:976; Viari, et al., 1987, Biomed. Environ. Mass Spectrom. 14:83; Grotjahn et al., 1982, Nuc. Acid Res. 10:4671). Sequencing methods are also available for RNA oligonucleotides.

The quality of oligonucleotides synthesized can be verified by testing the oligonucleotide by capillary electrophoresis and denaturing strong anion HPLC (SAX-HPLC) using, *e.g*., the method of Bergot and Egan. 1992. J. Chrom. 599:35.

Other exemplary synthesis techniques are well known in the art (see, *e.g*., Sambrook et al., Molecular Cloning: a Laboratory Manual, Second Edition (1989); DNA Cloning, Volumes I and II (DN Glover Ed. 1985); Oligonucleotide Synthesis (MJ Gait Ed, 1984; Nucleic Acid Hybridisation (BD Hames and SJ Higgins eds. 1984); A Practical Guide to Molecular Cloning (1984); or the series, Methods in Enzymology (Academic Press, Inc.)).

### Uses of Oligonucleotides

This invention also features methods wherein introduction of a double stranded nucleic acid molecule inhibits expression of a protein in a cell

The oligonucleotides can be used in a variety of *in vitro* situations to specifically inhibit protein expression.

The methods of the invention may be used for determining the function of a gene in a cell or an organism or for modulating the function of a gene in a cell or an organism, being capable of responding to or mediating RNA interference. The cell is a eukaryotic cell or a cell line, *e.g.,* an animal cell such as a mammalian cell, *e.g.,* an embryonic cell, a pluripotent stem cell, a tumor cell, *e.g.,* a teratocarcinoma cell, or a virus-infected cell. The organism is a eukaryotic organism, *e.g.,* an animal such as a mammal, particularly a human.

The invention includes methods to inhibit expression of a target gene in a cell *in vitro.* For example, such methods may include introduction of RNA into a cell in an amount sufficient to inhibit expression of the target gene, where the RNA is a double-stranded molecule of the invention. By way of a further example, such an RNA molecule may have a first strand consisting essentially of a ribonucleotide sequence that corresponds to a nucleotide sequence of the target gene, and a second strand consisting essentially of a ribonucleotide sequence that is complementary to the nucleotide sequence of the target gene, in which the first and the second strands are separate complementary strands or are joined by a loop, and they hybridize to each other to form said double-stranded molecule, such that the duplex composition inhibits expression of the target gene. The duplex composition may include modified nucleomonomers as discussed above.

Described is also a method to inhibit expression of a target gene in an invertebrate organism. Such methods include providing an invertebrate organism containing a target cell that contains the target gene, in which the target cell is susceptible to RNA interference and the target gene is expressed in the target cell. Such methods further include contacting the invertebrate organism with an RNA composition. For example, the RNA may be a double-stranded molecule with a first strand consisting essentially of a ribonucleotide sequence that corresponds to a nucleotide sequence of the target gene and a second strand consisting essentially of a ribonucleotide sequence that is complementary to the nucleotide sequence of the target gene. In such cases, the first and the second ribonucleotide sequences may be separate complementary strands or joined by a loop, and they hybridize to each other to form the double-stranded molecule. Finally, such methods include a step of introducing the duplex RNA composition into the target cell to thereby inhibiting expression of the target gene.

In one embodiment, the nucleic acid molecule can be used to inhibit gene function *in vitro* in a method for identifying the functions of genes. In this manner, the transcription of genes that are identified, but for which no function has yet been shown, can be inhibited to thereby determine how the phenotype of a cell is changed when the gene is not transcribed. Such methods are useful for the validation of genes as targets for clinical treatment, *e.g.,* with oligonucleotides or with other therapies.

To determine the effect of a composition a variety of end points can be used. In addition to the assays described previously herein, for example, nucleic acid probes (*e.g*., in the form of arrays) can be used to evaluate transcription patterns produced by cells. Probes can also be used detect peptides, proteins, or protein domains, *e.g*., antibodies can be used to detect the expression of a particular protein. In yet another example, the function of a protein (*e.g*., enzymatic activity) can be measured. In yet another example the phenotype of a cell can be evaluated to determine whether or not a target protein is expressed. For example, the ability of a composition to affect a phenotype of a cell that is associated with cancer can be tested.

In one embodiment, one or more additional agents (*e.g*., activating agents, inducing agents, proliferation enhancing agents, tumor promoters) can be added to the cells.

In another example the methods of the invention can be used to monitor biochemical reactions such as, *e.g*., interactions of proteins, nucleic acids, small molecules, or the like, for example the efficiency or specificity of interactions between antigens and antibodies; or of receptors (such as purified receptors or receptors bound to cell membranes) and their ligands, agonists or antagonists; or of enzymes (such as proteases or kinases) and their substrates, or increases or decreases in the amount of substrate converted to a product; as well as many others. Such biochemical assays can be used to characterize properties of the probe or target, or as the basis of a screening assay. For example, to screen samples for the presence of particular proteases (*e.g*., proteases involved in blood clotting such as proteases Xa and VIIa), the samples can be assayed, for example using probes which are fluorogenic substrates specific for each protease of interest. If a target protease binds to and cleaves a substrate, the substrate will fluoresce, usually as a result, *e.g.,* of cleavage and separation between two energy transfer pairs, and the signal can be detected. In another example, to screen samples for the presence of a particular kinase(s) (*e.g.,* a tyrosine kinase), samples containing one or more kinases of interest can be assayed, *e.g.,* using probes are peptides which can be selectively phosphorylated by one of the kinases of interest. Using art-recognized, routinely determinable conditions, samples can be incubated with an array of substrates, in an appropriate buffer and with the necessary cofactors, for an empirically determined period of time. If necessary, reactions can be stopped, *e.g.,* by washing and the phosphorylated substrates can be detected by, for example, incubating them with detectable reagents such as, *e.g.,* fluorescein-labeled anti-phosphotyrosine or anti-phosphoserine antibodies and the signal can be detected.

In another embodiment, the methods of the invention can be used to screen for agents which modulate a pattern of gene expression. Arrays of oligonucleotides can be used, for example, to identify mRNA species whose pattern of expression from a set of genes is correlated with a particular physiological state or developmental stage, or with a disease condition ("correlative" genes, RNAs, or expression patterns). By the terms "correlate" or "correlative," it is meant that the synthesis pattern of RNA is associated with the physiological condition of a cell, but not necessarily that the expression of a given RNA is responsible for or is causative of a particular physiological state. For example, a small subset of mRNAs can be identified which are modulated (*e.g*., upregulated or downregulated) in cells which serve as a model for a particular disease state. This altered pattern of expression as compared to that in a normal cell, which does not exhibit a pathological phenotype, can serve as a indicator of the disease state ("indicator" or "correlative" genes, RNAs, or expression patterns).

Compositions which modulate the chosen indicator expression pattern (*e.g*., compared to control compositions comprising, for example oligonucleotides which comprise a nucleotide sequence which is the reverse of the oligonucleotide, or which contains mismatch bases) can indicate that a particular target gene is a potential target for therapeutic intervention. Moreover, such compositions may be useful as therapeutic agents to modulate expression patters of cells in an *in vitro* expression system or in *in vivo* therapy. As used herein, "modulate" means to cause to increase or decrease the amount or activity of a molecule or the like which is involved in a measurable reaction. In one embodiment, a series of cells (*e.g*., from a disease model) can be contacted with a series of agents (*e.g*., for a period of time ranging from about 10 minutes to about 48 hours or more) and, using routine, art-recognized methods (*e.g.*, commercially available kits), total RNA or mRNA extracts can be made. If it is desired to amplify the amount of RNA, standard procedures such as RT-PCR amplification can be used (*see*, *e.g*., Innis et al eds., (1996) PCR Protocols: A Guide to Methods in Amplification, Academic Press, New York). The extracts (or amplified products from them) can be allowed to contact (*e.g*., incubate with) probes for appropriate indicator RNAs, and those agents which are associated with a change in the indicator expression pattern can be identified.

Similarly, agents can be identified which modulate expression patterns associated with particular physiological states or developmental stages. Such agents can be man-made or naturally-occurring substances, including environmental factors such as substances involved in embryonic development or in regulating physiological reactions.

In one embodiment, the methods described herein can be performed in a "high throughput" manner, in which a large number of target genes (*e.g*., as many as about 1000 or more, depending on the particular format used) are assayed rapidly and concurrently. Further, many assay formats (*e.g*., plates or surfaces) can be processed at one time. For example, because the oligonucleotides do not need to be tested individually before incorporating them into a composition, they can be readily synthesized and large numbers of target genes can be tested at one time. For example, a large number of samples, each comprising a biological sample containing a target nucleic acid molecule (*e.g*., a cell) and a composition can be added to separate regions of an assay format and assays can be performed on each of the samples.

### Administration of Oligonucleotide Compositions

The optimal course of administration or delivery of the oligonucleotides may vary depending upon the desired result and/ or on the subject to be treated. As used herein "administration" refers to contacting cells with oligonucleotides and can be performed *in vitro.* The dosage of oligonucleotides may be adjusted to optimally reduce expression of a protein translated from a target nucleic acid molecule, *e.g.*, as measured by a readout of RNA stability or by a therapeutic response, without undue experimentation.

For example, expression of the protein encoded by the nucleic acid target can be measured to determine whether or not the dosage regimen needs to be adjusted accordingly. In addition, an increase or decrease in RNA or protein levels in a cell or produced by a cell can be measured using any art recognized technique. By determining whether transcription has been decreased, the effectiveness of the oligonucleotide in inducing the cleavage of a target RNA can be determined.

Any of the above-described oligonucleotide compositions can be used alone or in conjunction with a pharmaceutically acceptable carrier. As used herein, "pharmaceutically acceptable carrier" includes appropriate solvents, dispersion media, coatings, antibacterial and antifungal agents, isotonic and absorption delaying agents, and the like. The use of such media and agents for pharmaceutical active substances is well known in the art. Except insofar as any conventional media or agent is incompatible with the active ingredient, it can be used in the therapeutic compositions. Supplementary active ingredients can also be incorporated into the compositions.

Oligonucleotides may be incorporated into liposomes or liposomes modified with polyethylene glycol or admixed with cationic lipids for parenteral administration. Incorporation of additional substances into the liposome, for example, antibodies reactive against membrane proteins found on specific target cells, can help target the oligonucleotides to specific cell types.

Moreover, the present description provides for administering the subject oligonucleotides with an osmotic pump providing continuous infusion of such oligonucleotides, for example, as described in Rataiczak et al. (1992 Proc. Natl. Acad Sci. USA 89:11823-11827). Such osmotic pumps are commercially available, *e.g.,* from Alzet Inc. (Palo Alto, Calif.). Topical administration in a cationic lipid carrier are preferred.

Drug delivery vehicles can be chosen *e.g*., for *in vitro,* administration. These vehicles can be designed to serve as a slow release reservoir or to deliver their contents directly to the target cell. An advantage of using some direct delivery drug vehicles is that multiple molecules are delivered per uptake. Such vehicles have been shown to increase the circulation half-life of drugs that would otherwise be rapidly cleared from the blood stream. Some examples of such specialized drug delivery vehicles which fall into this category are liposomes, hydrogels, cyclodextrins, biodegradable nanocapsules, and bioadhesive microspheres.

The chosen method of delivery will result in entry into cells. Preferred delivery methods include liposomes (10-400 nm), hydrogels, controlled-release polymers, and other pharmaceutically applicable vehicles, and microinjection or electroporation (for *ex vivo* treatments).

Examples of naturally occurring emulsifiers that may be used in emulsion formulations include lanolin, beeswax, phosphatides, lecithin and acacia. Finely divided solids have also been used as good emulsifiers especially in combination with surfactants and in viscous preparations. Examples of finely divided solids that may be used as emulsifiers include polar inorganic solids, such as heavy metal hydroxides, nonswelling clays such as bentonite, attapulgite, hectorite, kaolin, montmorillonite, colloidal aluminum silicate and colloidal magnesium aluminum silicate, pigments and nonpolar solids such as carbon or glyceryl tristearate.

Examples of preservatives that may be included in the emulsion formulations include methyl paraben, propyl paraben, quaternary ammonium salts, benzalkonium chloride, esters of p-hydroxybenzoic acid, and boric acid. Examples of antioxidants that may be included in the emulsion formulations include free radical scavengers such as tocopherols, alkyl gallates, butylated hydroxyanisole, butylated hydroxytoluene, or reducing agents such as ascorbic acid and sodium metabisulfite, and antioxidant synergists such as citric acid, tartaric acid, and lecithin.

In one example, the compositions of oligonucleotides are formulated as microemulsions. A microemulsion is a system of water, oil and amphiphile which is a single optically isotropic and thermodynamically stable liquid solution. Typically microemulsions are prepared by first dispersing an oil in an aqueous surfactant solution and then adding a sufficient amount of a 4th component, generally an intermediate chain-length alcohol to form a transparent system.

Surfactants that may be used in the preparation of microemulsions include, but are not limited to, ionic surfactants, non-ionic surfactants, Brij 96, polyoxyethylene oleyl ethers, polyglycerol fatty acid esters, tetraglycerol monolaurate (ML310), tetraglycerol monooleate (MO310), hexaglycerol monooleate (P0310), hexaglycerol pentaoleate (PO500), decaglycerol monocaprate (MCA750), decaglycerol monooleate (MO750), decaglycerol sequioleate (S0750), decaglycerol decaoleate (DA0750), alone or in combination with cosurfactants. The cosurfactant, usually a short-chain alcohol such as ethanol, 1-propanol, and 1-butanol, serves to increase the interfacial fluidity by penetrating into the surfactant film and consequently creating a disordered film because of the void space generated among surfactant molecules.

Microemulsions may, however, be prepared without the use of cosurfactants and alcohol-free self-emulsifying microemulsion systems are known in the art. The aqueous phase may typically be, but is not limited to, water, an aqueous solution of the drug, glycerol, PEG300, PEG400, polyglycerols, propylene glycols, and derivatives of ethylene glycol. The oil phase may include, but is not limited to, materials such as Captex 300, Captex 355, Capmul MCM, fatty acid esters, medium chain (C₈-C₁₂) mono, di, and tri-glycerides, polyoxyethylated glyceryl fatty acid esters, fatty alcohols, polyglycolized glycerides, saturated polyglycolized C₈-C₁₀ glycerides, vegetable oils and silicone oil.

Microemulsions are particularly of interest from the standpoint of drug solubilization and the enhanced absorption of drugs. Lipid based microemulsions (both oil/water and water/oil) have been proposed to enhance the oral bioavailability of drugs.

Microemulsions offer improved drug solubilization, protection of drug from enzymatic hydrolysis, possible enhancement of drug absorption due to surfactant-induced alterations in membrane fluidity and permeability, ease of preparation, ease of oral administration over solid dosage forms, improved clinical potency, and decreased toxicity (Constantinides et al., Pharmaceutical Research, 1994, 11:1385; Ho et al., J. Pharm. Sci., 1996, 85:138-143). Microemulsions have also been effective in the transdermal delivery of active components in both cosmetic and pharmaceutical applications. It is expected that the microemulsion compositions and formulations will facilitate the increased systemic absorption of oligonucleotides from the gastrointestinal tract, as well as improve the local cellular uptake of oligonucleotides within the gastrointestinal tract, vagina, buccal cavity and other areas of administration.

In an example various penetration enhancers to effect the efficient delivery of nucleic acids, particularly oligonucleotides, to the skin of animals may be used. Even non-lipophilic drugs may cross cell membranes if the membrane to be crossed is treated with a penetration enhancer. In addition to increasing the diffusion of non-lipophilic drugs across cell membranes, penetration enhancers also act to enhance the permeability of lipophilic drugs.

Five categories of penetration enhancers that may be used in the present invention include: surfactants, fatty acids, bile salts, chelating agents, and non-chelating non-surfactants. Other agents may be utilized to enhance the penetration of the administered oligonucleotides include: glycols such as ethylene glycol and propylene glycol, pyrrols such as 2-15 pyrrol, azones, and terpenes such as limonene, and menthone.

The oligonucleotides, especially in lipid formulations, can also be administered by coating a medical device, for example, a catheter, such as an angioplasty balloon catheter, with a cationic lipid formulation. Coating may be achieved, for example, by dipping the medical device into a lipid formulation or a mixture of a lipid formulation and a suitable solvent, for example, an aqueous-based buffer, an aqueous solvent, ethanol, methylene chloride, chloroform and the like. An amount of the formulation will naturally adhere to the surface of the device which is subsequently administered to a patient, as appropriate. Alternatively, a lyophilized mixture of a lipid formulation may be specifically bound to the surface of the device. Such binding techniques are described, for example, in K. Ishihara et al., Journal of Biomedical Materials Research, Vol. 27, pp. 1309-1314 (1993), the disclosures of which are incorporated herein by reference in their entirety.

The useful dosage to be administered and the particular mode of administration will vary depending upon such factors as the cell type, the particular oligonucleotide and delivery method used, the therapeutic or diagnostic use contemplated, and the form of the formulation, for example, suspension, emulsion, micelle or liposome, as will be readily apparent to those skilled in the art. Typically, dosage is administered at lower levels and increased until the desired effect is achieved. When lipids are used to deliver the oligonucleotides, the amount of lipid compound that is administered can vary and generally depends upon the amount of oligonucleotide agent being administered. For example, the weight ratio of lipid compound to oligonucleotide agent is preferably from about 1:1 to about 15:1, with a weight ratio of about 5:1 to about 10:1 being more preferred. Generally, the amount of cationic lipid compound which is administered will vary from between about 0.1 milligram (mg) to about 1 gram (g).

The agents of the invention are contacted with cells in a biologically compatible form suitable for pharmaceutical administration. By "biologically compatible form suitable for administration " is meant that the oligonucleotide is administered in a form in which any toxic effects are outweighed by the therapeutic effects of the oligonucleotide.

Administration of an active amount of an oligonucleotide is defined as an amount effective, at dosages and for periods of time necessary to achieve the desired result. For example, an active amount of an oligonucleotide may vary according to factors such as the type of cell, the oligonucleotide used, and the ability of the oligonucleotide to elicit a desired response. Establishment of therapeutic levels of oligonucleotides within the cell is dependent upon the rates of uptake and efflux or degradation. Decreasing the degree of degradation prolongs the intracellular half-life of the oligonucleotide. Thus, chemically-modified oligonucleotides, *e.g*., with modification of the phosphate backbone, may require different dosing.

The exact dosage of an oligonucleotide and number of doses administered will depend upon the data generated experimentally. Several factors such as the desired effect, the delivery vehicle, disease indication, and the route of administration, will affect the dosage. Dosages can be readily determined by one of ordinary skill in the art and formulated into the subject pharmaceutical compositions. Preferably, the duration of treatment will extend at least through the course of the disease symptoms.

Dosage regima may be adjusted to provide the optimum therapeutic response. For example, the oligonucleotide may be repeatedly administered, *e.g*., several doses may be administered daily or the dose may be proportionally reduced as indicated by the exigencies of the therapeutic situation. One of ordinary skill in the art will readily be able to determine appropriate doses and schedules of administration of the subject oligonucleotides.

### Treatment of Diseases or Disorders

By inhibiting the expression of a gene, the oligonucleotide compositions can be used to treat any disease involving the expression of a protein. Examples of diseases that can be treated by oligonucleotide compositions include: cancer, retinopathies, autoimmune diseases, inflammatory diseases (*i.e*., ICAM-1 related disorders, Psoriasis, Ulcerative Colitus, Crohn's disease), viral diseases (*i.e*., HIV, Hepatitis C), and cardiovascular diseases.

In one example, *in vitro* treatment of cells with oligonucleotides can be used for *ex vivo* therapy of cells removed from a subject (*e.g*., for treatment of leukemia or viral infection) or for treatment of cells which did not originate in the subject, but are to be administered to the subject (*e.g.*, to eliminate transplantation antigen expression on cells to be transplanted into a subject). In addition, *in vitro* treatment of cells can be used in non-therapeutic settings, *e.g.,* to evaluate gene function, to study gene regulation and protein synthesis or to evaluate improvements made to oligonucleotides designed to modulate gene expression or protein synthesis. There are numerous medical conditions for which antisense therapy is reported to be suitable (*see*, *e.g*., U.S. 5,830,653) as well as respiratory syncytial virus infection (WO 95/22,553) influenza virus (WO 94/23,028), and malignancies (WO 94/08,003). Other examples of clinical uses of antisense sequences are reviewed, *e.g.,* in Glaser. 1996. Genetic Engineering News 16:1. Exemplary targets for cleavage by oligonucleotides include, e.g., protein kinase Ca, ICAM-1, c-raf kinase, p53, c-myb, and the bcr/abl fusion gene found in chronic myelogenous leukemia.

The practice of the present invention will employ, unless otherwise indicated, conventional techniques of cell biology, cell culture, molecular biology, microbiology, recombinant DNA, and immunology, which are within the skill of the art. Such techniques are explained fully in the literature. *See,* for example, Molecular Cloning A Laboratory Manual, 2nd Ed., ed. by Sambrook, J. et al. (Cold Spring Harbor Laboratory Press (1989)); Short Protocols in Molecular Biology, 3rd Ed., ed. by Ausubel, F. et al. (Wiley, NY (1995)); DNA Cloning, Volumes I and II (D. N. Glover ed., 1985); Oligonucleotide Synthesis (M. J. Gait ed. (1984)); Mullis et al. U.S. Patent No: 4,683,195; Nucleic Acid Hybridization (B. D. Hames & S. J. Higgins eds. (1984)); the treatise, Methods In Enzymology (Academic Press, Inc., N.Y.); Immunochemical Methods In Cell And Molecular Biology (Mayer and Walker, eds., Academic Press, London (1987)); Handbook Of Experimental Immunology, Volumes I-IV (D. M. Weir and C. C. Blackwell, eds. (1986)); and Miller, J. Experiments in Molecular Genetics (Cold Spring Harbor Press, Cold Spring Harbor, N.Y. (1972)).

The invention is further illustrated by the following examples, which should not be construed as further limiting.

### EXAMPLES

### Example 1. Oligonucleotide Compositions Comprising Chimeric Antisense Sequences.

A gapped antisense oligonucleotide comprising 2'-O-methyl RNA arms and an unmodified DNA gap was synthesized. A complementary oligonucleotide was also synthesized using unmodified RNA. A double-stranded duplex was formed and the composition was found to inhibit expression of the target gene.

### Example 2. Length of Double-Stranded Oligonucleotides and the Presence or Absence of Overhangs Has No Effect on Function.

Twenty one and 27-mers were designed to target each of two sites on the p53 molecule (89-90 site, and 93-94 site). The double-stranded molecules were designed with or without 3'-deoxy TT overhangs. The test oligonucleotides were 21-mers with 2 nucleotide 3' deoxy TT overhangs and without overhangs (blunt ends); and 27-mers with 2 nucleotide 3' deoxy TT overhangs and without overhangs (blunt ends). Two positive controls were included in the experiment (p53) and two negative controls were also included (FITC)

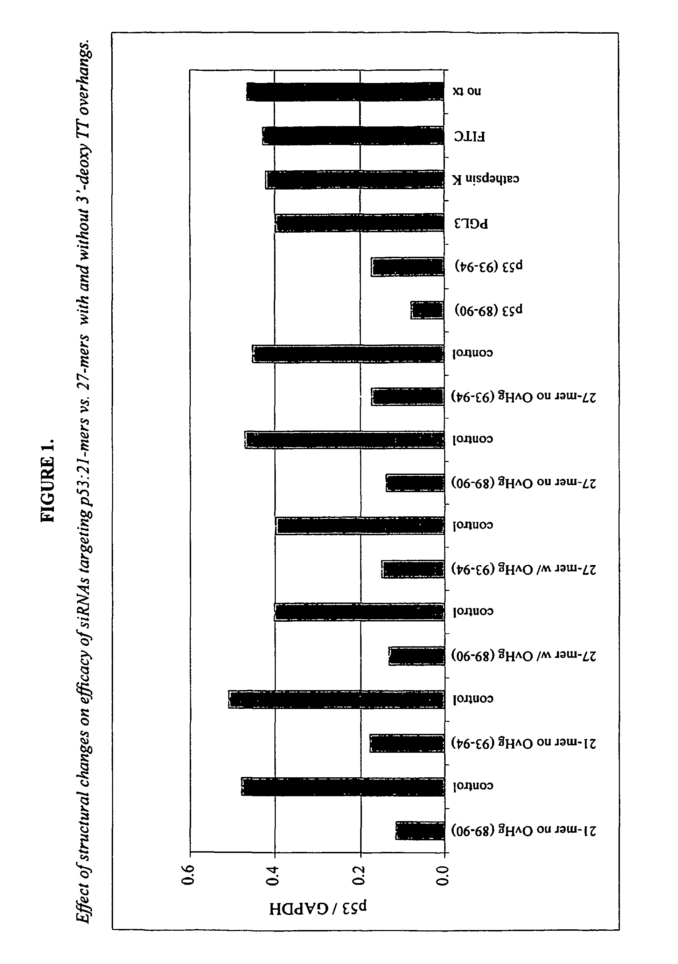

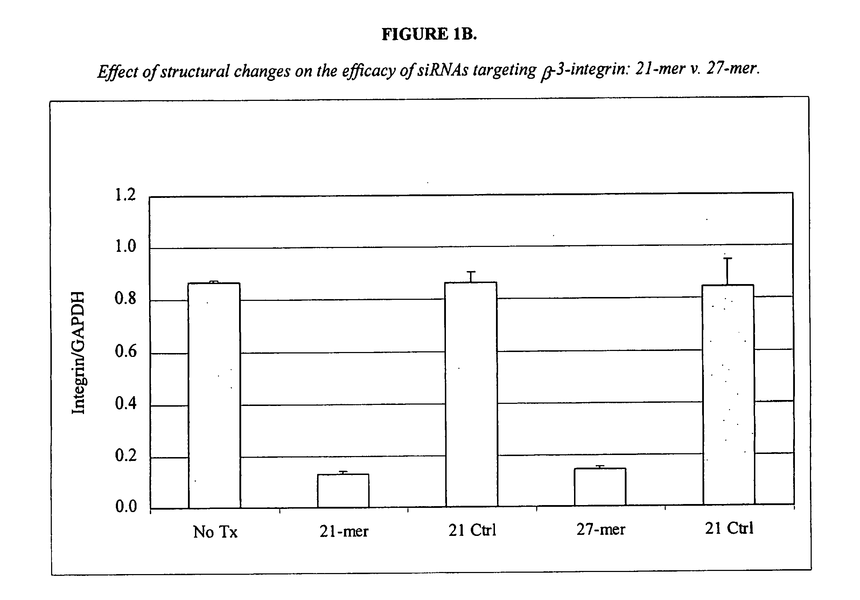

A549 cells were transfected with 100 nM of the double-stranded molecules plus 2 ug/mL Lipofectamine 2000. A549 cells were examined 24 hours post-transfection. FITC-labeled molecules were taken up well by cells. Both 21-mers (with or without overhangs) and 27-mers (with or without overhangs) were non-toxic to cells. Figure 1 shows the result of an experiment comparing the ability of different oligonucleotide constructs to inhibit p53 and shows that length or the presence or absence of a 3' deoxy TT overhang did not affect the activity of the oligonucleotide. The results in Figure 1 show the amount of p53 mRNA normalized to the amount of an irrelevant message, GAPDH. The level of mRNA was determined using RT-PCR analysis. The observed percent inhibition of p53 expression is shown below:

| SITE | | 21-MER | | 27-MER |
|---|---|---|---|---|
| | overhang | no overhang | overhang | no overhang |
| 93-94 | 58% | 65% | 62% | 62% |
| 89-90 | 81% | 75% | 67% | 70% |

Similar results were observed for β-3-integrin; both 21-mer and 27-mer double-stranded molecules were found to inhibit integrin mRNA. Two double-stranded RNA complexes designed to target the same site of the β-3-integrin gene were transfected in HMVEC cells. Both complexes contained a two nucleotide (TT) overhang: one complex was a 21-mer (with 19 nucleotides complementary to the target gene) and the other was a 27-mer (with 25 nucleotides complementary to the target gene). RT-PCR analysis showed that the two complexes inhibited the target gene to the same extent. HMVEC cells were transfected using 100 nM oligomer complexed with 2ug/mL of Lipofecatmine 2000 in media containing serum for 24 hours. Twenty-four hours after transfection, the cells were lysed and the RNA was isolated for analysis by RT-PCR. No significant toxicity was observed. The results in Figure 1B show the amount of β-3-integrin mRNA normalized to the amount of GAPDH, as determined by RT-PCR analysis.

### Example 3. Activation of the Double-Stranded RNA, Interferon-Inducible Protein Kinase, PKR.

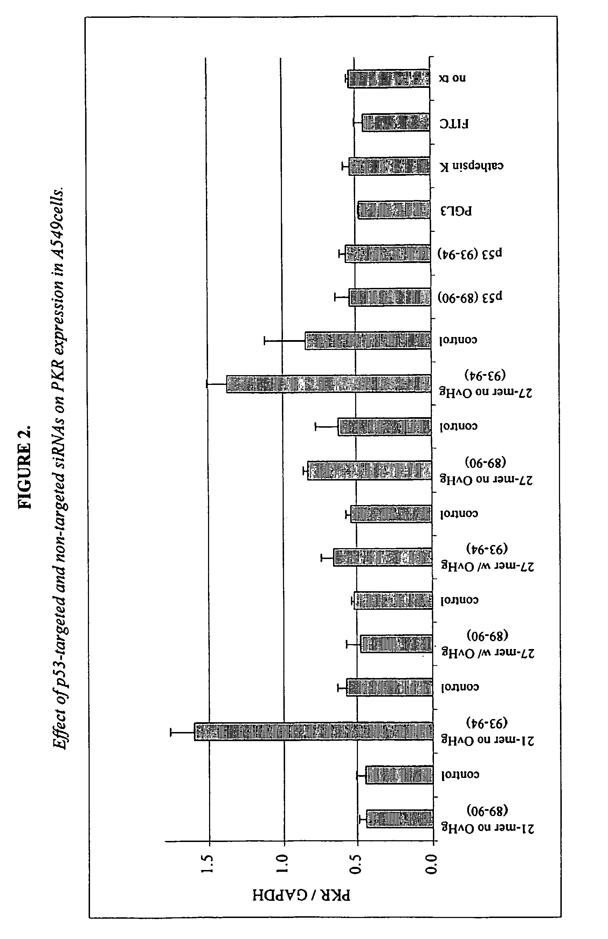

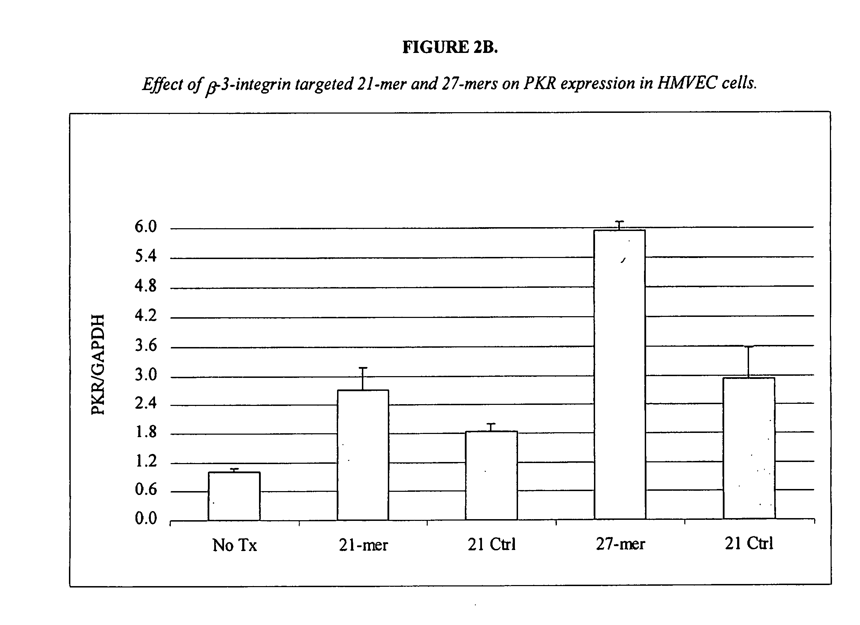

PKR is activated by double-stranded RNA molecules. Active PKR leads to the inhibition of protein synthesis, activation of transcription, and a variety of other cellular effects, including signal transduction, cell differentiation, cell growth inhibition, apoptosis, and antiviral effects. The effect of p53-targetd double-stranded RNA molecules on PKR expression was tested. The level of mRNA was determined using RT-PCR analysis. As shown in Figure 2, no correlation was observed between the length of the double-stranded oligonucleotide and the level of PKR induction. Accordingly, long oligonucleotides can be used without activating PKR, a marker for interferon induction.

As illustrated in Figure 2B, analysis of relative amounts of PKR mRNA after the 21-and 27-mer transfection in HMVEC cells showed approximately a 2 fold increase in PKR mRNA of the siRNA control sequences over no treatment, and approximately a 2 fold increase of PKR mRNA of the 27-mer compared to the 21-mer targeted double-stranded RNA complexes.

### Example 4. The Effect of 5' vs. 3' Modification on the Activity of Double-Stranded Oligonucleotides.

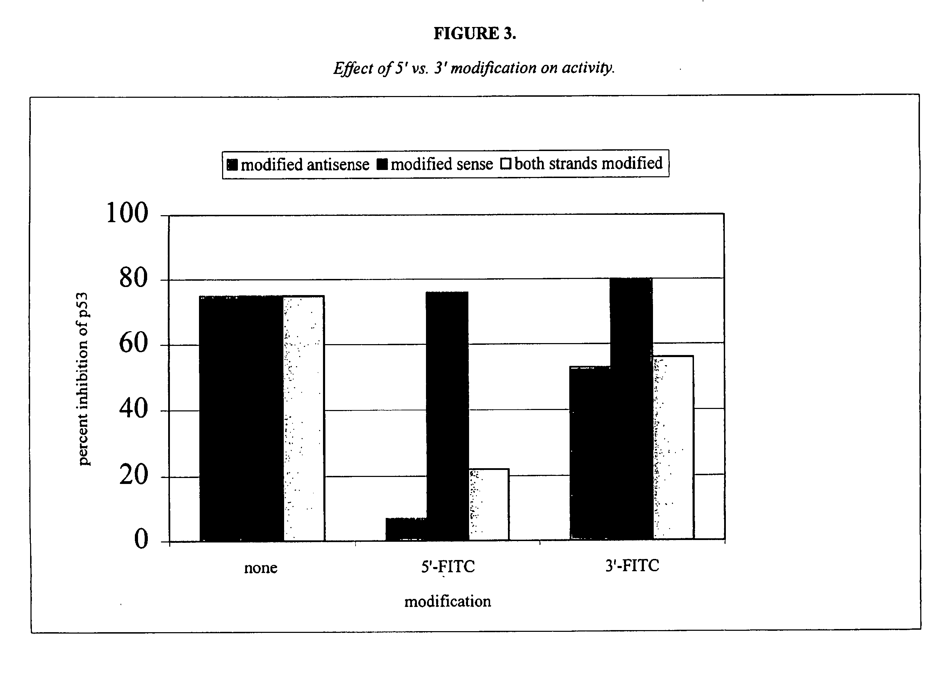

Oligonucleotide duplexes were modified at either the 3' or 5' end with FITC groups. The modifications were made on either the antisense strand or the sense strand. 5' or 3' modification of the sense strand had no effect on the percent inhibition of p53 mRNA. 3' modification of the antisense strand had little affect on activity, while 5' modification of the antisense strand reduced activity significantly. 3' modification of both strands also had little affect on activity, while 3' and 5' modification of both strands reduced activity. *See* Figure 3.

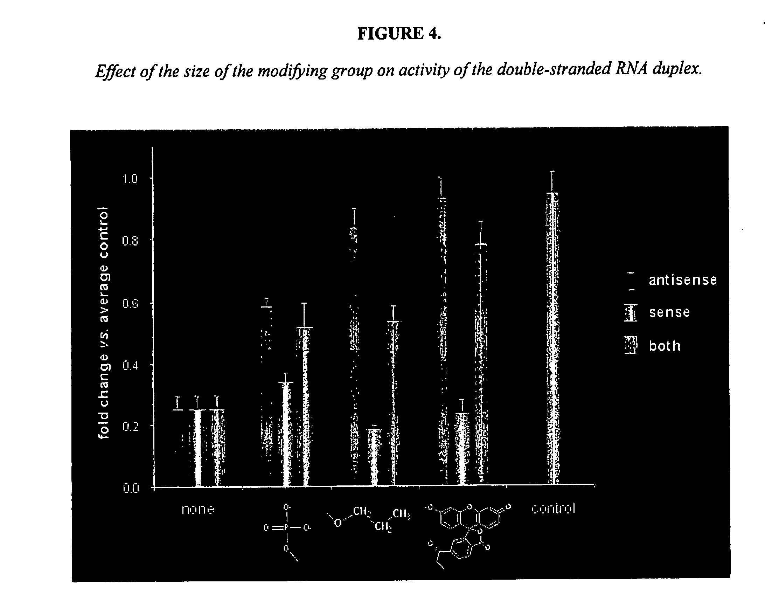

The effect of the size of the group used to modify the 5' end was tested. The results of this experiment are shown in Figure 4. The inclusion of a 5' phosphate group had little affect on activity, whereas the modification of the antisense strand or both strands had a greater effect. The inclusion of a propyl group had more of an effect, with a 5' propyl group on the antisense strand showing a large reduction in activity; there was also an effect when this group was added to both strands. Similarly, the inclusion of a FITC group at the 5' end of the antisense molecule (or to both molecules) also significantly reduced the activity of the RNA duplex.

### Example 5. Comparison of the Efficacy of 2'-O-Me Modified and Unmodified Double-Stranded RNA Oligonucleotides.

A549 cells were transfected with modified or unmodified RNA duplexes complexed at 100 nM with 2 ug/mL Lipofectamine 2000 (Invitrogen) and were transfected for 24 hours. The A549 cells were plated at 20,000/well in 48 well plates. After 24 hours, FITC-labeled double-stranded oligonucleotides were visible in A549 cells; the inclusion of a 2'-O-Me group did not affect uptake. The Table below shows the results of this experiment.

| 2'-O-Me Oligonucleotide Duplexes | | | | |
|---|---|---|---|---|
| | Anti-sense/Sense | Anti-sense/Sense | Anti-sense/Sense | Anti-sense/Sense |
| | 2'-O-Me/2'-OMe | 2'-O-Me/RNA | RNA/2'-O-Me | RNA/RNA |
| targeted | 18639/18640 | 18639/16194 | 16193/18640 | 18876 |
| non-targeted | 19039/19040 | 19039/19044 | 19043/19040 | 18850 & 16197/16198 |
| | | | | |
| | FITC-2'-O-Me/FITC 2'-O-Me | FITC-2'-O-Me/FITC-RNA | FITC-2'-O-Me/RNA | 2'-O-Me/FITC-RNA |
| non-targeted | 19209 | 19037/19042 | 19037/19044 | 19039/19042 |

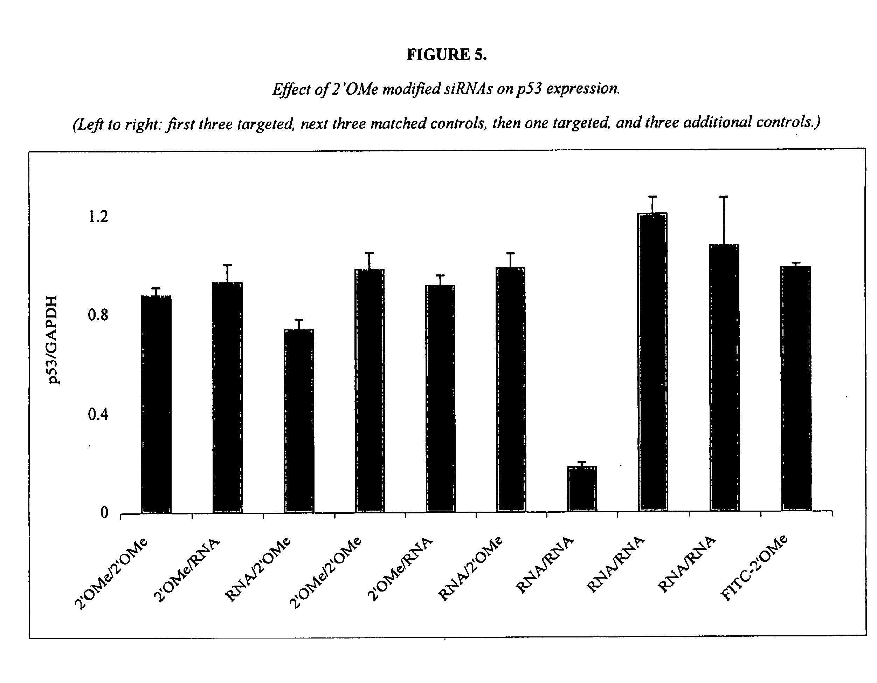

The affect of 2'-O-Me modifications to one or both strands of a double-stranded RNA molecule is shown in Figure 5.

### Example 6. Toxicity of p53-Targeted siRNAs in A549 Cells.

27-mer siRNAs targeting p53 were not toxic to cells when compared to standard 21-mer siRNAs having 3' deoxy TT overhangs. In this experiment, both siRNA constructs inhibited p53 to a similar extent (83% inhibition for 27-mer vs. 90% inhibition for 21-mer). siRNAs were designed to target p53 and were constructed as blunt-end 27-mers or as 21-mers with 3' deoxy TT overhangs. A549 cells were plated at 20,000 cells per well in 48-well plates on the day prior to transfection. On the day of transfection, cells were approximately 60-70% confluent. Cells were transfected with 100 nM siRNAs complexed with 2 ug / mL Lipofectamine 2000 for 24 hours. Following transfection, cells were stained with Dead Red stain to visualize the extent of cell death. The siRNA sequences used were as follows:

| 21-mer with overhangs targeted (5'-3'): | |
|---|---|
| ACCUCAAAGCUGUUCCGUCTT | (SEQ ID NO: ##) |
| GACGGAACAGCUUUGAGGUTT | (SEQ ID NO: ##) |
| | |

| Blunt-end 27-mer targeted (5'-3'): | |
|---|---|
| ACGCACACCUCAAAGCUGUUCCGUCCC | (SEQ ID NO: ##) |
| GGGACGGAACAGCTTTGAGGTGTGCGT | (SEQ ID NO: ##) |

### Example 7. Toxicity of Blunt-End 27-mer siRNAs Targeting p53 in A549 Cells.

The toxicity of targeted blunt-end 27-mer siRNAs targeting p53 was observed to be not significantly different than a control nucleic acid or no treatment. siRNAs were designed to target p53 and were constructed as blunt-end 27-mers. The corresponding control consisted of chemistry-matched, scrambled sequences with a similar base-pair composition. A549 cells were plated at 20,000 cells per well in 48-well plates on the day prior to transfection. On the day of transfection, cells were approximately 60-70% confluent. Cells were transfected with 100 nM siRNAs complexed with 2 ug / mL Lipofectamine 2000 for 24 hours. Following transfection, the cells were stained with Dead Red stain to visualize the extent of cell death. the siRNA sequences used were as follows:

| Blunt-end 27-mer targeted (5'-3' on top): | |
|---|---|
| ACGCACACCUCAAAGCUGUUCCGUCCC | (SEQ ID NO: ##) |
| GGGACGGAACAGCTTTGAGGTGTGCGT | (SEQ ID NO: ##) |
| | |

| Corresponding control (5'-3' on top): | |
|---|---|
| CCCTGCCTTGTCGAAACTCCACACGCA | (SEQ ID NO: ##) |
| TGCGTGTGGAGTTTCGACAAGGCAGGG | (SEQ ID NO: ##) |

### Example 8. Toxicity of Blunt End 32-mer siRNAs Targeting p53 in A549 Cells.

Similarly, blunt-end 32-mer siRNAs targeting p53 were not observed to be toxic to cells in comparison with a control nucleic acid and no treatment, as determined by Dead Red staining. siRNAs were designed to target p53 and were constructed as blunt-end 32-mers. The corresponding control consisted of chemistry-matched, scrambled sequences with a similar base-pair composition. A549 cells were plated at 20,000 cells per well in 48-well plates on the day prior to transfection. On the day of transfection, cells were approximately 60-70% confluent. Cells were transfected with 100 nM siRNAs complexed with 2 ug / mL Lipofectamine 2000 for 24 hours. Following transfection, cells were stained with Dead Red stain to visualize the extent of cell death. The siRNA sequences used were as follows:

| Targeted blunt-end 32-mer (5'-3' on top:) | |
|---|---|
| CCCTCACGCACACCUCAAAGCUGUUCCGUCCC | (SEQ ID NO: ##) |
| GGGACGGAACAGCTTTGAGGTGTGCGTGAGGG | (SEQ ID NO: ##) |
| | |

| Corresponding control (5'-3' on top): | |
|---|---|
| CCCTGCCTTGTCGAAACTCCACACGCACTCCC | (SEQ ID NO: ##) |
| GGGAGTGCGTGTGGAGTTTCGACAAGGCAGGG | (SEQ ID NO: ##) |

### Example 9. Inhibition of p53 by 32- and 37-mer Blunt-End siRNAs.

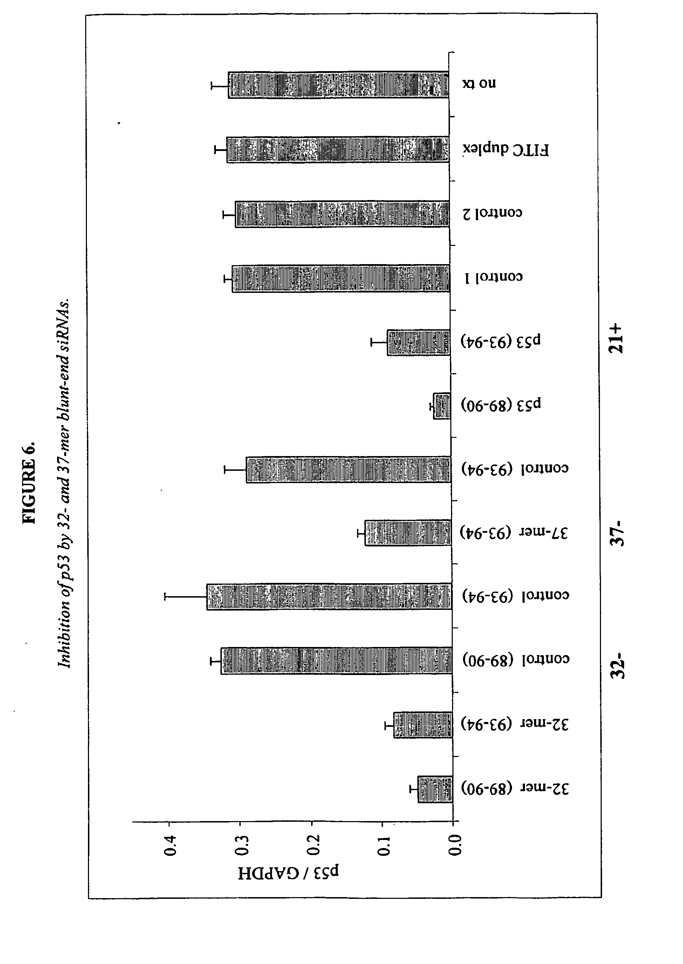

Figure 6 depicts the results of inhibition of p53 by 32- and 37-mer blunt-end siRNAs in comparison with various control experiments. siRNAs were designed to target each of two sites (93-93 site) and (89-90 site) along the coding region of p53. siRNAs were constructed as blunt-end 32-mers or blunt-end 37-mers. Positive control siRNAs were 21-mers with 3' deoxy TT overhangs. Corresponding controls consisted of chemistry-matched, scrambled sequences with a similar base-pair composition. A549 cells were plated at 20,000 cells per well in 48-well plates on the day prior to transfection. On the day of transfection, cells were approximately 60-70% confluent. Cells were transfected with 100 nM siRNAs complexed with 2 ug / mL Lipofectamine 2000 for 24 hours. Following transfection, cells were lysed and poly(A) mRNA was harvested for RT-PCR. Inhibition of p53 expression was determined by quantitative real-time RT-PCR (TaqMan) analysis. Expression of p53 was standardized by quantifying GAPDH for each sample. The data in Figure 6 represent three separate transfections analyzed in duplicate and normalized to the internal control (GAPDH). The siRNA sequences used were as follows (depicted with the 5'-3' strand on top):

| Targeted 32-mer (89-90 site): | |
|---|---|
| CCCTCACGCACACCUCAAAGCUGUUCCGUCCC | (SEQ ID NO: ##) |
| GGGACGGAACAGCTTTGAGGTGTGCGTGAGGG | (SEQ ID NO: ##) |
| | |

| 32-mer control (89-90 site): | |
|---|---|
| CCCTGCCTTGTCGAAACTCCACACGCACTCCC | (SEQ ID NO: ##) |
| GGGAGTGCGTGTGGAGTTTCGACAAGGCAGGG | (SEQ ID NO: ##) |
| | |

| 32-mer targeted (93-94 site): | |
|---|---|
| CCCUUCUGUCUUGAACAUGAGTTTTTTATGGC | (SEQ ID NO: ##) |
| GCCATAAAAAACTCATGTTCAAGACAGAAGGG | (SEQ ID NO: ##) |
| | |

| 32-mer control (93-94 site): | |
|---|---|
| CGGTATTTTTTGAGTACAAGTTCTGTCTTCCC | (SEQ ID NO: ##) |
| GGGAAGACAGAACTTGTACTCAAAAAATACCG | (SEQ ID NO: ##) |
| | |

| 37-mer targeted (93-94 site): | |
|---|---|
| CCCTTCTGTCTTGAACATGAGTTTTTTATGGCGGGAG | (SEQ ID NO: ##) |
| CTCCCGCCATAAAAAACTCATGTTCAAGACAGAAGGG | (SEQ ID NO: ##) |

| 37-mer control (93-94 site): | |
|---|---|
| GAGGGCGGTATTTTTTGAGTACAAGTTCTGTCTTCCC | (SEQ ID NO: ##) |
| GGGAAGACAGAACTTGTACTCAAAAAATACCGCCCTC | (SEQ ID NO: ##) |
| | |

| 21-mer targeted (89-90 site): | |
|---|---|
| ACCUCAAAGCUGUUCCGUCTT | (SEQ ID NO: ##) |
| GACGGAACAGCUUUGAGGUTT | (SEQ ID NO: ##) |
| | |

| 21-mer targeted (93-94 site): | |
|---|---|
| CCCUUCUGUCUUGAACAUGTT | (SEQ ID NO: ##) |
| CAUGUUCAAGACAGAAGGGTT | (SEQ ID NO: ##) |

### Example 10. Enhanced Cellular Stability of Double-Stranded 2'-O-Methyl RNA.

In this example, the single-stranded control oligomer was transfected at 800 nM. Accumulation was observed in the nucleus at 6 hours post transfection, however by 25 hours the fluorescence of the single-stranded oligomer had largely dissipated, indicating the oligomer was no longer intact (Fisher, T., T. Terhorst, et al. (1993). "Intracellular disposition and metabolism of fluorescently-labeled unmodifieed and modified oligonucleotides microinjected into mammalian cells." NAR 21: 3857-3865). The relative fluorescence of fluorescently-labeled oligomers transfected into A549 cells was observed to fit the following pattern:

| | single-stranded (800 nM) | double-stranded (100 nm) |
|---|---|---|
| 6 h | ++++ | +++++ |
| 25 h | + | +++++ |

The double-stranded oligomer duplex, wherein the second strand was 2'-O-methyl modified RNA, was transfected at 100nM, and was also clearly visible at 6 hours post transfection. However, in contrast to the single-stranded oligomer, the double-stranded was still largely intact in the nucleus at 24 hours, even though the concentration transfected was 8-fold less, thereby demonstrating that the 2'-O-methyl second strand stabilized the oligomer in the cell.

The oligomers were all 2'-O-CH₃ with a phosphodiester backbone containing 6-carboxyfluorescein (6-FAM) tethered to the 5' hydroxyl. The single-stranded control oligomer was transfected at 800nM complexed with 4 ug/mL of Lipofectamine 2000, and the double-stranded complex was transfected at 100 nM complexed with 1 ug/mL of Lipofectamine 2000.

Fluorescent signal was seen accumulating in the nucleus at 6 hours post transfection, however by 24 hours the single-stranded oligomer has significantly dissipated, indicating the oligomer is no longer intact. The double-stranded duplexes (wherein the second strand is 2'-O-methyl modified RNA with a 5' 6-FAM) was transfected at 100nM, and was also clearly visible at 6 hours post transfection. In contrast to the single-stranded oligomer, the double-stranded was still largely intact in the nucleus at 24 hours, even though the concentration transfected was 8-fold less. This experiment demonstrates that the 2'-O-methyl second strand stabilizes the duplex in the cell.

### Example 11. Enhanced Stability in Cells and Accumulation in Cytoplasm of RNA Hybridized to 2'-O-Methyl RNA.

The fluorescence signal, corresponding to uptake of FITC-labeled RNA and 2'-O-methyl modified RNA duplexes, was measured at 6 and 24 hours. RNA complexes were transfected in A549 cells with 100 nM oligomer complexed with 2ug/mL Lipofecatmine 2000 as described below. Cells were continuously transfected for 24 hours and fluorescent uptake was assessed at 6 and 24 hours. Oligomers were 2'-O-methyl modified RNA with 5' 6-FAM (FITC-2'-OMe), 19-mer RNA with two deoxynucleotides on the 3' end with 5' 6-FAM (FITC-RNA) or 19-mer RNA with two deoxynucleotides on the 3' end (RNA) complexed. At 6 hours, the FITC-2'-O-methyl duplexes show localization in the nucleus and the FITC-2'-O-methyl/RNA and 2'-O-methyl/FITC-RNA complexes show a more diffuse pattern of uptake (these RNA/2'-O-methyl complexes are a substrate for the RISC complex and are therefore retained in the cytoplasm where the RISC complex has been reported to be active). At 24 hours, the FITC-2'-O-methyl/RNA and 2'-O-methyl/FITC-RNA complexes were still visible in the cell, whereas typically not even the single-stranded FITC-2'-O- was visible, even when transfected at significantly higher concentrations, demonstrating that the 2'-O-methyl RNA protects the RNA strand from degradation in the cell.

RNA oligomers having a phosphodiester backbone with 2'-O-methyl nucleotides were synthesized using standard phosphoramidite chemistry. Oligomers were purified by denaturing polyacrylamide gel electrophoresis (PAGE). Purity of oligomers was confirmed by (PAGE) and mass spectrometry. All oligomers were greater than 90% full length, and mass data obtained was consistent with expected values. Target-specific siRNA duplexes consisted of 21-nt sense and 21-nt antisense strands with symmetric 2-nt 3' deoxy TT overhangs. 21-nt RNAs were chemically synthesized using phosphoramidite chemistry. For duplex preparation, sense- and antisense oligomers (each at 50 µM) were combined in equal volumes in annealing buffer (30 mM HEPES pH 7.0, 100 mM potassium acetate, and 2 mM magnesium acetate), heat-denatured at 90 °C for 1 min and annealed at 37 °C for one hour. Duplexes were stored at 80 °C until used.

A549 cells (ATCC #CCL-185) were cultured at 37 °C in Dulbecco's Modified Eagle Medium (DMEM, Life Technologies #11960-044) supplemented with 2 mM L-glutamine, 100 units / mL penicillin, 100 µg / mL streptomycin, and 10% fetal bovine serum (FBS). HeLa cells (ATCC #CCL-2) were cultured at 37 °C in Minimal Essential Medium (MEM, Life Technologies #10370-021) supplemented with 2 mM L-glutamine, 1.5 g / L sodium bicarbonate, 1.0 mM sodium pyruvate, 100 units / mL penicillin, 100 µg / mL streptomycin, and 10% FBS. Cells were passaged regularly to maintain exponential growth. On the day prior to transfection, cells were trypsinized, counted, and seeded in 48-well plates at a density of 20 x 103 cells per well in 250 µL fresh media. On the day of transfection cells were typically 60-65% confluent. Transfection of siRNA duplexes and oligomers was carried out using Lipofectamine 2000 (Life Technologies). Briefly, a 10X stock of Lipofectamine 2000 was prepared in Opti-Mem (Life Technologies) and incubated at room temperature for 15 minutes. An equal volume of a 10X stock of siRNA duplex or oligomers in Opti-Mem was added and complexation carried out for 15 minutes at room temperature. Complexes were then diluted 5-fold in full growth media. Culture media was removed from each well prior to the addition of 250 µL complexes per well. Cells were incubated at 37 °C/5% CO₂ for 6 or 24 hours prior to assessing the uptake.

### Equivalents

Those skilled in the art will recognize, or be able to ascertain using no more than routine experimentation, many equivalents to the specific embodiments of the invention described herein. Such equivalents are intended to be encompassed by the following claims. The entire contents of all patents, published patent applications and other references cited herein are hereby expressly incorporated herein in their entireties by reference.

## Claims

1. A method for introducing a double-stranded nucleic acid molecule into a eukaryotic cell *in vitro,* the method comprising contacting the eukaryotic cell with the double-stranded nucleic acid molecule which comprises a first nucleotide sequence and a second nucleotide sequence which are of equal length;
wherein at least one of the first nucleotide sequence or of the second nucleotide sequence contains at least four 5'-terminal 2'-O-methyl nucleotides, and
wherein the double-stranded nucleic acid molecule is between 18 and 30 nucleotides in length.

2. The method of claim 1, wherein the double-stranded nucleic acid molecule is introduced into the eukaryotic cell by electroporation.

3. The method of claim 1, wherein a strand of said double-stranded nucleic acid molecule is complementary to a sequence of an mRNA expressed in said eukaryotic cell.

4. The method of claim 3, wherein introduction of said double-stranded nucleic acid molecule in said eukaryotic cell inhibits expression of protein from said mRNA.

5. The method of claim 3, wherein said double-stranded nucleic acid molecule is a double-stranded RNA molecule.

6. The method of claim 5, wherein introduction of said double-stranded RNA molecule in said eukaryotic cell inhibits expression of protein from said mRNA.

7. The method of Claim 1, wherein the number of nucleotides in each of the first nucleotide sequence and the second nucleotide sequence of the double-stranded nucleic acid molecule is 21.

8. The method of Claim 1, wherein the number of nucleotides in each of the first nucleotide sequence and the second nucleotide sequence of the double-stranded nucleic acid molecule is 27.

9. The method of Claim 1, wherein the number of nucleotides in each of the first nucleotide sequence and the second nucleotide sequence of the double-stranded nucleic acid molecule is 25.

10. The method of Claim 1, wherein the first nucleotide sequence of the double-stranded nucleic acid molecule contains at least four 5' terminal 2'-O-methyl nucleotides.

11. The method of Claim 1, wherein the second nucleotide sequence of the double-stranded nucleic acid molecule contains at least four 5' terminal 2'-O-methyl nucleotides.

12. The method of Claim 1, wherein the first nucleotide sequence and the second nucleotide sequence of the double-stranded nucleic acid molecule contains at least four 5' terminal 2'-O-methyl nucleotides.

## Patentansprüche

1. Verfahren zum In-vitro-Einbringen eines doppelsträngigen Nukleinsäuremoleküls in eine Eukayrotenzelle, wobei in dem Verfahren die Eukaryotenzelle mit dem doppelsträngigen Nukleinsäuremolekül in Kontakt gebracht wird, das eine erste Nukleotidsequenz und eine zweite Nukleotidsequenz mit gleicher Länge aufweist;
wobei zumindest eine der ersten Nukleotidsequenz und der zweiten Nukleotidsequenz zumindest vier 2'-O-Methyl-Nukleotide am 5'-Ende enthält, und
wobei das doppelsträngige Nukleinsäuremolekül eine Länge zwischen 18 und 30 Nukleotiden aufweist.

2. Verfahren nach Anspruch 1, wobei das doppelsträngige Nukleinsäuremolekül durch Elektroporation in die Eukaryotenzelle eingebracht wird.

3. Verfahren nach Anspruch 1, wobei ein Strang des doppelsträngigen Nukleinsäuremoleküls mit einer Sequenz einer in der Eukaryotenzelle exprimierten mRNA komplementär ist.

4. Verfahren nach Anspruch 3, wobei das Einbringen des doppelsträngigen Nukleinsäuremoleküls in die Eukaryotenzelle die Exprimierung von Proteinen aus der mRNA hemmt.

5. Verfahren nach Anspruch 3, wobei das doppelsträngige Nukleinsäuremolekül ein doppelsträngiges RNA-Molekül ist.

6. Verfahren nach Anspruch 5, wobei das Einbringen des doppelsträngigen RNA-Moleküls in die Eukaryotenzelle die Exprimierung von Proteinen aus der mRNA hemmt.

7. Verfahren nach Anspruch 1, wobei die Anzahl der Nukleotide in jeder der ersten Nukleotidsequenz und der zweiten Nukleotidsequenz des doppelsträngigen Nukleinsäuremoleküls 21 beträgt.

8. Verfahren nach Anspruch 1, wobei die Anzahl der Nukleotide in jeder der ersten Nukleotidsequenz und der zweiten Nukleotidsequenz des doppelsträngigen Nukleinsäuremoleküls 27 beträgt.

9. Verfahren nach Anspruch 1, wobei die Anzahl der Nukleotide in jeder der ersten Nukleotidsequenz und der zweiten Nukleotidsequenz des doppelsträngigen Nukleinsäuremoleküls 25 beträgt.

10. Verfahren nach Anspruch 1, wobei die erste Nukleotidsequenz des doppelsträngigen Nukleinsäuremoleküls zumindest vier 2'-O-Methyl-Nukleotide am 5'-Ende enthält.

11. Verfahren nach Anspruch 1, wobei die zweite Nukleotidsequenz des doppelsträngigen Nukleinsäuremoleküls zumindest vier 2'-O-Methyl-Nukleotide am 5'-Ende enthält.

12. Verfahren nach Anspruch 1, wobei die erste Nukleotidsequenz und die zweite Nukleotidsequenz des doppelsträngigen Nukleinsäuremoleküls zumindest vier 2'-O-Methyl-Nukleotide am 5'-Ende enthalten.

## Revendications

1. Procédé d'introduction d'une molécule d'acide nucléique à double brin dans une cellule eucaryote *in vitro,* le procédé comprenant la mise en contact de la cellule eucaryote avec la molécule d'acide nucléique à double brin qui comprend une première séquence nucléotidique et une seconde séquence nucléotidique qui sont de longueur équivalente ;
dans lequel au moins une de la première séquence nucléotidique ou de la seconde séquence nucléotidique contient au moins quatre 2'-O-méthyl nucléotides à l'extrémité 5', et
dans lequel la molécule d'acide nucléique à double brin a une longueur comprise entre 18 à 30 nucléotides.

2. Procédé selon la revendication 1, dans lequel la molécule d'acide nucléique à double brin est introduite dans la cellule eucaryote par électroporation.

3. Procédé selon la revendication 1, dans lequel un brin de ladite molécule d'acide nucléique à double brin est complémentaire à une séquence d'un ARNm exprimé dans ladite cellule eucaryote.

4. Procédé selon la revendication 3, dans lequel l'introduction de ladite molécule d'acide nucléique à double brin dans ladite cellule eucaryote inhibe l'expression de la protéine à partir dudit ARNm.

5. Procédé selon la revendication 3, dans lequel ladite molécule d'acide nucléique à double brin est une molécule d'ARN à double brin.

6. Procédé selon la revendication 5, dans lequel l'introduction de ladite molécule d'ARN à double brin dans ladite cellule eucaryote inhibe l'expression de la protéine à partir dudit ARNm.

7. Procédé selon la revendication 1, dans lequel le nombre de nucléotides dans chacune de la première séquence nucléotidique et de la seconde séquence nucléotidique de la molécule d'acide nucléique à double brin est de 21.

8. Procédé selon la revendication 1, dans lequel le nombre de nucléotides dans chacune de la première séquence nucléotidique et de la seconde séquence nucléotidique de la molécule d'acide nucléique à double brin est de 27.

9. Procédé selon la revendication 1, dans lequel le nombre de nucléotides dans chacune de la première séquence nucléotidique et de la seconde séquence nucléotidique de la molécule d'acide nucléique à double brin est de 25.

10. Procédé selon la revendication 1, dans lequel la première séquence nucléotidique de la molécule d'acide nucléique à double brin contient au moins quatre 2'-O-méthyl nucléotides à l'extrémité 5'.

11. Procédé selon la revendication 1, dans lequel la seconde séquence nucléotidique de la molécule d'acide nucléique à double brin contient au moins quatre 2'-O-méthyl nucléotides à l'extrémité 5'.

12. Procédé selon la revendication 1, dans lequel la première séquence nucléotidique et la seconde séquence nucléotidique de la molécule d'acide nucléique à double brin contient au moins quatre 2'-O-méthyl nucléotides à l'extrémité 5'.
